(19) **Europäisches Patentamt / European Patent Office / Office européen des brevets**

(11) **EP 2 864 915 B1**

(12) **EUROPEAN PATENT SPECIFICATION**

(45) Date of publication and mention of the grant of the patent:
**04.05.2022  Bulletin 2022/18**

(21) Application number: **13730567.8**

(22) Date of filing: **21.06.2013**

(51) International Patent Classification (IPC):
**G16B 5/00** *(2019.01)*

(52) Cooperative Patent Classification (CPC):
**G06N 20/10; G16B 5/00; G16B 5/30; G16B 40/00; G16B 40/20; G16B 40/30**

(86) International application number:
**PCT/EP2013/062979**

(87) International publication number:
**WO 2013/190083 (27.12.2013 Gazette 2013/52)**

(54) **SYSTEMS AND METHODS RELATING TO NETWORK-BASED BIOMARKER SIGNATURES**

SYSTEME UND VERFAHREN IM ZUSAMMENHANG MIT NETZWERKBASIERTEN BIOMARKERSIGNATUREN

SYSTÈMES ET PROCÉDÉS RELATIFS À DES SIGNATURES DE BIOMARQUEURS BASÉES SUR RÉSEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.06.2012  US 201261662806 P**
**16.07.2012  US 201261671954 P**

(43) Date of publication of application:
**29.04.2015  Bulletin 2015/18**

(73) Proprietors:
• **Philip Morris Products S.A.**
  **2000 Neuchâtel (CH)**
• **Martin, Florian**
  **2034 Peseux (CH)**
• **Sewer, Alain**
  **1350 Orbe (CH)**
• **Hoeng, Julia**
  **2035 Corcelles (CH)**
• **Peitsch, Manuel Claude**
  **2034 Peseux (CH)**

(72) Inventors:
• **MARTIN, Florian**
  **CH-2034 Peseux (CH)**
• **SEWER, Alain**
  **CH-1350 Orbe (CH)**
• **HOENG, Julia**
  **CH-2035 Corcelles (CH)**
• **PEITSCH, Manuel Claude**
  **CH-2034 Peseux (CH)**

(74) Representative: **Ponder, William Anthony John et al**
  **Reddie & Grose LLP**
  **The White Chapel Building**
  **10 Whitechapel High Street**
  **London E1 8QS (GB)**

(56) References cited:
• **ANONYMOUS: "Reverse Causal Reasoning Methods - White Paper", INTERNET CITATION, 4 February 2011 (2011-02-04), pages 1-26, XP002681944, Retrieved from the Internet: URL:http://www.selventa.com/attachments/white_papers/reverse-causal-reasoning.pdf [retrieved on 2012-08-17]**
• **Wei Keat Lim ET AL: "Master regulators used as breast cancer metastasis classifier", Pac Symp Biocomput. 2009, 1 January 2009 (2009-01-01), pages 504-515, XP055078924, Retrieved from the Internet: URL:http://www.ncbi.nlm.nih.gov/pmc/articles/PMC2740937/pdf/nihms106219.pdf [retrieved on 2013-09-12]**

**(Cont. next page)**

- JULIA HOENG ET AL: "A network-based approach to quantifying the impact of biologically active substances", DRUG DISCOVERY TODAY, vol. 17, no. 9-10, 1 May 2012 (2012-05-01) , pages 413-418, XP055071052, ISSN: 1359-6446, DOI: 10.1016/j.drudis.2011.11.008
- FLORIAN MARTIN ET AL: "Assessment of network perturbation amplitudes by applying high-throughput data to causal biological networks", BMC SYSTEMS BIOLOGY, BIOMED CENTRAL LTD, LO, vol. 6, no. 1, 31 May 2012 (2012-05-31), page 54, XP021116941, ISSN: 1752-0509, DOI: 10.1186/1752-0509-6-54
- Jason Weston ET AL: "Feature selection for SVMs", Advances in Neural Information Processing Systems 13, 1 January 2001 (2001-01-01), pages 668-674, XP055066674, Retrieved from the Internet: URL:http://www0.cs.ucl.ac.uk/staff/M.Pontil/reading/featsel.pdf [retrieved on 2013-06-13]
- JURJEN W WESTRA ET AL: "Construction of a computable cell proliferation network focused on non-diseased lung cells", BMC SYSTEMS BIOLOGY, vol. 5, no. 1, 1 January 2011 (2011-01-01) , pages 105-105, XP055029252, ISSN: 1752-0509, DOI: 10.1186/1752-0509-5-105

## Description

## Background

In the last decade, high-throughput measurements of nucleic acid, protein and metabolite levels in conjunction with traditional dose-dependent efficacy and toxicity assays, have emerged as a means for elucidating mechanisms of action of many biological processes. Researchers have attempted to combine information from these disparate measurements with knowledge about biological pathways from the scientific literature to assemble meaningful biological models. To this end, researchers have begun using mathematical and computational techniques that can mine large quantities of data, such as clustering and statistical methods, to identify possible biological mechanisms of action.

Finding gene signatures that are sufficiently reliable for diagnostic tools is very challenging due to the high signal-to-noise ratio in typical gene expression data, the genotypic variability across individuals, and the high number of genes that are typically measured relative to the number of patients. Previous work has explored the importance of uncovering a characteristic signature of gene expression changes that results from one or more perturbations to a biological process, and the subsequent scoring of the presence of that signature in additional data sets as a measure of the specific activity amplitude of that process. Most work in this regard has involved identifying and scoring signatures that are correlated with a disease phenotype. These phenotype-derived signatures provide significant classification power, but lack a mechanistic or causal relationship between a single specific perturbation and the signature. Consequently, these signatures may represent multiple distinct unknown perturbations that, by often unknown mechanism(s), lead to, or result from, the same disease phenotype.

One challenge lies in understanding how the activities of various individual biological entities in a biological system enable the activation or suppression of different biological mechanisms. Because an individual entity, such as a gene, may be involved in multiple biological processes (e.g., inflammation and cell proliferation), measurement of the activity of the gene is not sufficient to identify the underlying biological process that triggers the activity.

None of the current techniques has been applied to identify the underlying mechanisms responsible for the activity of biological entities on a micro-scale, nor provide a quantitative assessment of the activation of different biological mechanisms in which these entities play a role, in response to potentially harmful agents and experimental conditions. Accordingly, there is a need for improved systems and methods for analyzing system-wide biological data in view of biological mechanisms, and quantifying changes in the biological system as the system responds to an agent or a change in the environment.

The use of reverse causal reasoning as a tool for reducing large-scale molecular profiling data to testable mechanistic hypotheses, and evaluation of those hypotheses, is disclosed in: Reverse Causal Reasoning Methods - White Paper, pp. 1-26,2011 [online], retrieved from http://www.selventa.com on 17.08.2017. However, this document does not disclose the generation of a classifier for phenotype prediction.

A computational systems biology approach that can infer robust prognostic markers by identifying upstream Master Regulators, causally related to the presentation of the phenotype of interest is disclosed in: Wei Keat Kim et al. Master regulators used as breast cancer metastasis classifier, Pac Symp Biocomput. 504-15, 2009 However, the underlying network model is different from the present invention in that it does not use direct measurements for biological entities (e.g., gene expression values) to infer the activity of upstream regulators.

## Summary

The invention is defined in the appended independent claims. Preferred or advantageous features of the invention are defined in the dependent claims. Aspects, embodiments or examples falling outside the scope of the appended independent claims are not part of the invention, and are merely included for illustrative or explanatory purposes.

Described herein are systems, computer program products and methods for identifying biological entities (for example, genes and proteins) and their properties that are representative of a phenotype of interest. The systems, computer program products and methods are based on the measured activities of a plurality of biological entities and a network model of a biological system contributing to the phenotype of interest that describes the relationships between various biological entities in the biological system. These network-based approaches utilize causal biological network models, which represent knowledge of "cause-and-effect" mechanisms identified in the research literature and published data sets, among other data sources. For example, in some causal biological network models, changes in gene transcription are modeled as the consequence of other biological processes represented in the model. In some implementations, network models of biological systems are described using Biological Expression Language ("BEL"), an open-source framework for biological network representation developed by Selventa of Cambridge, Massachusetts. The network-based approaches described herein use high throughput data sets and causal biological network models to quantitatively evaluate the perturbation of biological networks within the samples (e.g., patients). In some implementations, this evaluation includes translating observed activity measures of biological entities within the network (e.g.,

expression levels of genes) into inferred activity values for other biological entities within the network. The measured and inferred activities of biological entities in the network may then be used to represent the correlation of biological events or mechanisms with phenotypes that are observed at the cell, tissue, or organ level. Activities and their accompanying statistics provide a quantifiable measure of the degree of changes or perturbation of a biological network relating to the phenotype of interest, and indicate how changes in the properties of biological entities in the network propagate through the network topology. The latter may aid in building knowledge-driven classifiers that achieve higher accuracy than known classifiers, thus providing a better generalization of the biological phenomena of interest. As described herein, the activity values may be used to identify from a list of biological entities a subset of entities that can serve as a biological signature that is biologically meaningful and interpretable, and in its usage as a diagnostic or prognostic tool, robust and efficient.

In some aspects, provided herein are computerized methods and systems for processing treatment data to identify biological entities that are representative of a phenotype of interest. A processing device provides a computational causal network model that represents a biological system that contributes to the phenotype. The computational causal network model includes a plurality of nodes that represent biological entities in the biological system. For example, the nodes may correspond to compounds, DNA, RNA, proteins, peptides, antibodies, cells, tissues, or organs. The network model also includes a plurality of edges connecting pairs of nodes among the plurality of nodes and representing relationships between the biological entities represented by the nodes. For example, edges may represent a "binds to" relation, an "is expressed in" relation, an "are co-regulated based on expression profiling" relation, an "inhibits" relation, a "co-occur in a manuscript" relation, or "share structural element" relation. In the computational causal network model, one or more edges is associated with a direction value that represents a causal activation or causal suppression relationship between the biological entities represented by the nodes, and each node is connected by an edge to at least one other node.

The processing device receives (i) a first set of data corresponding to activities of a first subset of biological entities obtained under a first set of conditions, and (ii) a second set of data corresponding to activities of the first subset of biological entities obtained under a second set of conditions different from the first set of conditions. For example, the first and second set of conditions may correspond to treatment and control data, respectively, and the activity measures include a fold-change, which is a number describing how much a node measurements changes from an initial value to a final value between control data and treatment data. The first and second sets of conditions relate to the phenotype. The processing device also calculates a set of activity measures for a first subset of nodes corresponding to the first subset of biological entities, the activity measures representing a difference between the first set of data and the second set of data. The activity measures may include a fold-change or a logarithm of the difference between the treatment and control data for the biological entity represented by the node.

The processing device generates a set of activity values for a second subset of nodes representing candidates of biological entities that contribute to the phenotype but whose activities are not measured, based on the computational causal network model and the set of activity measures. The second subset of nodes corresponds to backbone entities because these nodes are not measured directly. Instead, the activity values of the second subset of nodes are inferred from the first set of activity values and the computational network model. The processing device further generates, using a machine learning technique, a classifier for the phenotypes based on the set of activity values, the set of activity measures, or both.

In certain embodiments of the methods described above, the step of generating the classifier comprises generating an operator that translates information about the activity measures of the first subset of biological entities into information about the activity values for the second subset of nodes, using the operator to identify a subset of the second subset of nodes, and providing the identified subset as an input to the machine learning technique. The operator corresponds to a backbone operator that acts on a vector of activity measures of a set of supporting nodes (i.e., the first subset of biological entities) and provides a vector of activity values for a set of backbone nodes (i.e., the second subset of nodes). Furthermore, multiple backbone operators may be combined via a weighted average or a non-linear function. For example, multiple backbone operators may be combined via a kernel alignment technique, and the backbone operators may be aggregated using significance values of one or more perturbations tests.

In certain embodiments of the methods described above, the calculating step of the set of activity measures and the generating step of the set of activity values steps are performed for a plurality of computational causal network models. The resulting plurality of sets of activity values corresponding to each of the computational causal network models are aggregated into the set of activity values used at the step of generating the classifier. In certain embodiments of the methods described above, the calculating step of the set of activity measures, the generating step of the set of activity values, and the generating step of the classifier are performed for a plurality of computational causal network models. The method further comprises identifying, for each classifier, one or more biological entities of the second set of biological entities with classification performance statistics above a threshold and aggregating all of the identified biological entities into a set of high performing entities. The processing device generates a new classifier of biological conditions based on the activity values associated with the set of high performing entities using a machine learning technique and outputs the new classifier. The high performing entities may correspond to an aggregate set of backbone nodes across multiple network models, each backbone node in the aggregate set being associated with an above-threshold value.

In certain embodiments of the methods described above, the machine learning technique includes a support vector machine technique. In certain embodiments of the methods described above, the generating step of the set of activity values comprises identifying, for each particular node in the second subset of nodes, an activity value that minimizes a difference statement. The difference statement represents the difference between the activity value of the particular node and the activity value or activity measure of nodes to which the particular node is connected by an edge within the computational causal network model, and the difference statement depends on the activity values of each node in the second subset of nodes. In certain embodiments of the methods described above, the difference statement further depends on the direction values of each node in the second subset of nodes. The difference statement may correspond to an expression or an executable statement that represents the difference between the activity measure or activity value of a particular biological entity and the activity measure or activity value of biological entities to which the particular biological entity is connected. In particular, the difference statement represents the difference between the activity measure or value of a particular node in a network model and the activity measure or value of nodes to which the particular node is connected via an edge.

In certain embodiments of the methods described above, each activity value in the set of activity values is a linear combination of activity measures in the set of activity measures. In certain embodiments of the methods described above, the linear combination depends on edges between nodes in the first subset of nodes and nodes in the second subset of nodes, and also depends on edges between nodes in the second subset of nodes. In certain embodiments of the methods described above, the linear combination does not depend on edges between nodes in the first subset of nodes. In certain embodiments of the methods described above, the method further comprises providing a variation estimate for each activity value of the set of activity values by forming a linear combination of variation estimates for each activity measure of the set of activity measures. In certain embodiments of the methods described above, the activity measure of the calculating step is a fold-change value, and the fold-change value for each node represents a logarithm of the difference between corresponding sets of treatment data for the biological entity represented by the respective node. In certain embodiments of the methods described above, the first subset of biological entities includes a set of genes and the first set of data include expression levels of the set of genes.

The computer program product and the computerized methods described herein may be implemented in a computerized system having one or more computing devices, each including one or more processors. Generally, the computerized systems described herein may comprise one or more engines, which include a processing device or devices, such as a computer, microprocessor, logic device or other device or processor that is configured with hardware, firmware, and software to carry out one or more of the computerized methods described herein. Any one or more of these engines may be physically separable from any one or more other engines, or may include multiple physically separable components, such as separate processors on common or different circuit boards. The computer systems of the present disclosure comprises means for implementing the methods and its various embodiments as described above. In certain implementations, the computerized system includes a systems response profile engine, a network modeling engine, and a network scoring engine. The engines may be interconnected from time to time, and further connected from time to time to one or more databases, including a perturbations database, a measurables database, an experimental data database and a literature database. The computerized system described herein may include a distributed computerized system having one or more processors and engines that communicate through a network interface. Such an implementation may be appropriate for distributed computing over multiple communication systems.

## Brief Description Of The Drawings

Further features of the disclosure, its nature and various advantages, will be apparent upon consideration of the following detailed description, taken in conjunction with the accompanying drawings, in which like reference characters refer to like parts throughout, and in which:

FIG. 1 is a block diagram of an illustrative computerized system for quantifying the response of a biological network to a perturbation.

FIG. 2 is a flow diagram of an illustrative process for generating a gene signature based on quantifying the response of one or more relevant biological network(s) to a perturbation.

FIG. 3 is a graphical representation of data underlying a systems response profile comprising data for two agents, two parameters, and N biological entities.

FIG. 4 is an illustration of a computational model of a biological network having several biological entities (nodes) and their relationships (edges which are directional and signed).

FIG. 5 is a flow diagram of an illustrative process for quantifying the perturbation of a biological system by calculating network perturbation amplitude (NPA).

FIG. 6 is a flow diagram of an illustrative process for generating activity values for a set of nodes.

FIG. 7 is a flow diagram of an illustrative process for identifying leading backbone and gene nodes.

FIG. 8 is a flow diagram of an illustrative process for classifying backbone node activity values.

FIG. 9 is a flow diagram of an illustrative process for identifying a feature space from multiple networks for use in identifying entities for biomarkers.

FIG. 10 is a flow diagram of an illustrative process for identifying a feature space from multiple classifiers for use in identifying entities for biomarkers.

FIG. 11 is a flow diagram of an illustrative process for identifying backbone nodes for use in a classification system based on F-statistics.

FIG. 12 is a flow diagram of an illustrative process for generating an ensemble predictor from backbone node activity values.

FIG. 13 is a flow diagram of an illustrative process for identifying backbone nodes for use in a classification system based on p-values.

FIG. 14 is a block diagram of an exemplary distributed computerized system for quantifying the impact of biological perturbations.

FIG. 15 is a block diagram of an exemplary computing device which may be used to implement any of the components in any of the computerized systems described herein.

FIG. 16 illustrates a causal biological network model with backbone nodes and supporting nodes.

FIG. 17 illustrates the leading node identification techniques of FIGS. 7 and 8.

FIG. 18 illustrates the multiple-network feature space identification techniques of FIGS. 9 and 10.

FIG. 19 is a graph depicting NPA scores for various treatment/control conditions using a TNF-IL1-NF$_K$B network model.

FIG. 20 illustrates a leading backbone node list for the TNF-IL1-NF$_K$B network model.

## Detailed Description

Described herein are computational systems and methods that assess quantitatively the magnitude of changes within a biological system when it is perturbed by an agent. Certain implementations include methods for computing a numerical value that expresses the magnitude of changes within a portion of a biological system. The computation uses as input, a set of data obtained from a set of controlled experiments or clinical data in which the biological system is perturbed by an agent. The data is then applied to a network model of a feature of the biological system. The network model is used as a substrate for simulation and analysis, and is representative of the biological mechanisms and pathways that enable a feature of interest in the biological system. The feature or some of its mechanisms and pathways may contribute to the pathology of diseases and adverse effects of the biological system. Prior knowledge of the biological system represented in a database is used to construct the network model which is populated by data on the status of numerous biological entities under various conditions including under normal conditions, disease conditions, and under perturbation by an agent. The network model used is a causal biological network model and is dynamic in that it represents changes in status of various biological entities underlying a disease or in response to a perturbation, and can yield quantitative and objective assessments of the changes associated with a disease or the impact of an agent on the biological system, including predictions of the behavior of biological entities "upstream" from measured gene expression levels. Computer systems for executing these computational methods are also provided.

The numerical values generated by computerized methods of the disclosure can be used to determine the magnitude of desirable or adverse biological effects that are associated with a disease or its symptoms, caused by manufactured products (for safety assessment or comparisons), therapeutic compounds including nutrition supplements (for determination of efficacy or health benefits), and environmentally active substances (for prediction of risks of long term exposure and the relationship to adverse effect and onset of disease), among others. The numerical values may also be used to predict phenotypic properties of a patient based on clinical data (e.g., predicting whether a patient will be responsive to a drug).

In one aspect, the systems and methods described herein provide a computed numerical value representative of the magnitude of change in a perturbed biological system based on a network model of a perturbed biological mechanism. The numerical value referred to herein as a network perturbation amplitude (NPA) score can be used to summarily represent the status changes of various entities in a defined biological mechanism. The numerical values obtained for different agents or different types of perturbations can be used to compare relatively the impact of the different agents or various perturbations associated with the onset or development of a disease on a biological mechanism which enables or manifests itself as a feature of a biological system. Thus, NPA scores may be used to measure the responses of a biological mechanism to different perturbations. The term "score" is used herein generally to refer to a value or set of values which provide a quantitative measure of the magnitude of changes in a biological system. Such a score is computed by using any of various mathematical and computational algorithms known in the art and according to the methods disclosed herein, employing one or more datasets obtained from a sample or a subject.

The NPA scores may assist researchers and clinicians in improving diagnosis, experimental design, therapeutic decision, and risk assessment. For example, the NPA scores may be used to screen a set of candidate biological mechanisms in a toxicology analysis to identify those most likely to be affected by exposure to a potentially harmful agent. By providing a measure of network response to a perturbation, these NPA scores may allow correlation of molecular events (as measured by experimental data) with phenotypes or biological outcomes that occur at the cell, tissue, organ or organism level. A clinician may use NPA values to compare the biological mechanisms affected by an agent to a patient's physiological condition to determine what health risks or benefits the patient is most likely to experience when exposed to the agent (e.g., a patient who is immuno-compromised may be especially vulnerable to agents that cause a strong immuno-suppressive response).

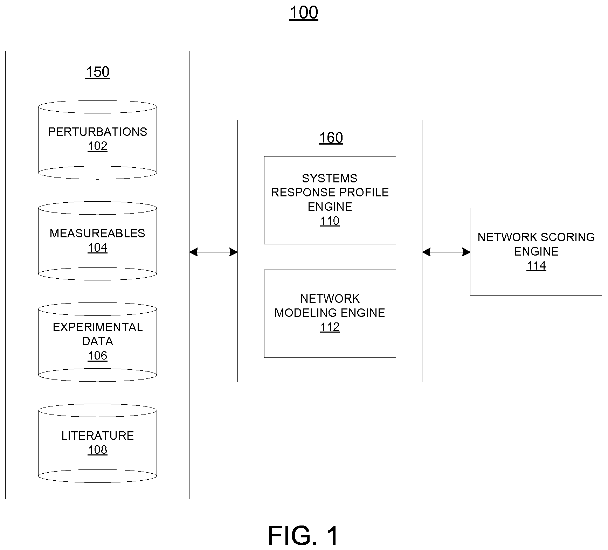

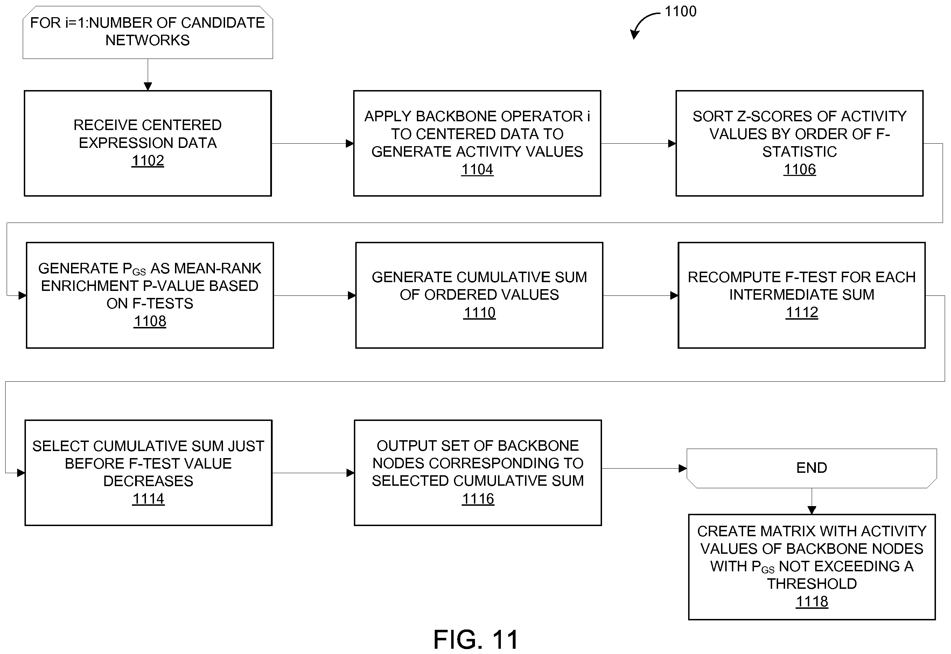

FIG. 1 is a block diagram of a computerized system 100 for quantifying the response of a network model to a perturbation. In particular, system 100 includes a systems response profile engine 110, a network modeling engine 112, and a network scoring engine 114. The engines 110, 112, and 114 are interconnected from time to time, and further connected from time to time to one or more databases, including a perturbations database 102, a measurables database 104, an experimental data database 106 and a literature database 108. As used herein, an engine includes a processing device or devices, such as a computer, microprocessor, logic device or other device or devices as described with reference to FIG. 11, configured with hardware, firmware, and software to carry out one or more computational operations.

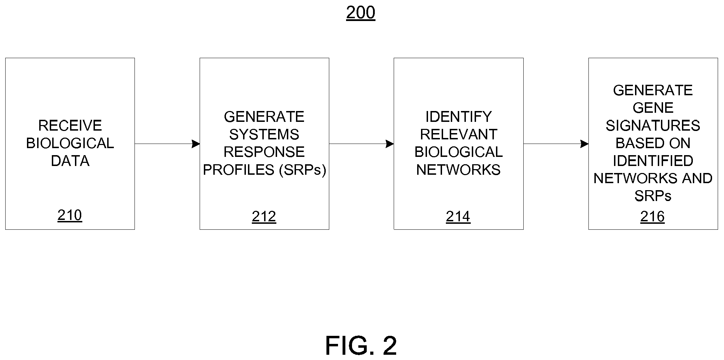

FIG. 2 is a flow diagram of a process 200 for generating a network signature or a gene signature that is based on quantifying the response of a biological network to a perturbation by calculating a network perturbation amplitude (NPA) score, according to one implementation. The steps of the process 200 will be described as being carried out by various components of the system 100 of FIG. 1, but any of these steps may be performed by any suitable hardware or software components, local or remote, and may be arranged in any appropriate order or performed in parallel. At step 210, the systems response profile (SRP) engine 110 receives biological data from a variety of different sources, and the data itself may be of a variety of different types. The data includes clinical data, epidemiology data, and data from experiments in which a biological system is perturbed, as well as control data. At step 212, the SRP engine 110 generates systems response profiles (SRPs) which are representations of known or unrecognized pathological changes associated with a disease, or the degree to which one or more entities within a biological system change in response to the presentation of an agent to the biological system. At step 214, the network modeling engine 112 provides one or more databases that contain(s) a plurality of network models, one of which is selected as being relevant to a disease, the agent or a feature of interest. The selection can be made on the basis of prior knowledge of the mechanisms underlying the biological functions of the system. In certain implementations, the network modeling engine 112 may extract causal relationships between entities within the system using the systems response profiles, networks in the database, and networks previously described in the literature, thereby generating, refining or extending a network model. At step 216, the network scoring engine 114 generates NPA scores for each perturbation using the network identified at step 214 by the network modeling engine 112 and the SRPs generated at step 212 by the SRP engine 110. An NPA score quantifies a biological response to a perturbation or treatment (represented by the SRPs) in the context of the underlying relationships between the biological entities (represented by the network). The following description is divided into subsections for clarity of disclosure, and not by way of limitation.

A biological system in the context of the present disclosure is an organism or a part of an organism, including functional parts, the organism being referred to herein as a subject. The subject is generally a mammal, including a human. The subject can be an individual human being in a human population. The term "mammal" as used herein includes but is not limited to a human, non-human primate, mouse, rat, dog, cat, cow, sheep, horse, and pig. Mammals other than humans can be advantageously used as subjects that can be used to provide a model of a human disease. The non-human subject can be unmodified, or a genetically modified animal (e.g., a transgenic animal, or an animal carrying one or more genetic mutation(s), or silenced gene(s)). A subject can be male or female. Depending on the objective of the operation, a subject can be one that has been exposed to an agent of interest. A subject can be one that has been exposed to an agent over an extended period of time, optionally including time prior to the study. A subject can be one that had been exposed to an agent for a period of time but is no longer in contact with the agent. A subject can be one that has been diagnosed or identified as having a disease. A subject can be one that has already undergone, or is undergoing treatment of a disease or adverse health condition. A subject can also be one that exhibits one or more symptoms or risk factors for a specific health condition or disease. A subject can be one that is predisposed to a disease, and may be either symptomatic or asymptomatic. In certain implementations, the disease or health condition in question is associated with exposure to an agent or use of an agent over an extended period of time. According to some implementations, the system 100 (FIG. 1) contains or generates computerized models of one or more biological systems and mechanisms of its functions (collectively, "biological networks" or "network models") that are relevant to a type of perturbation or an outcome of interest.

Depending on the context of the operation, the biological system can be defined at different levels as it relates to the function of an individual organism in a population, an organism generally, an organ, a tissue, a cell type, an organelle, a cellular component, or a specific individual's cell(s). Each biological system comprises one or more biological mechanisms or pathways, the operation of which manifest as functional features of the system. Animal systems that reproduce defined features of a human health condition and that are suitable for exposure to an agent of interest are preferred biological systems. Cellular and organotypical systems that reflect the cell types and tissue involved in a disease etiology or pathology are also preferred biological systems. Priority could be given to primary cells or organ cultures that recapitulate as much as possible the human biology *in vivo.* It is also important to match the human cell culture *in vitro* with the most equivalent culture derived from the animal models *in vivo.* This enables creation of a translational continuum from animal model to human biology *in vivo* using the matched systems *in vitro* as reference systems. Accordingly, the biological system contemplated for use with the systems and methods described herein can be defined by, without limitation, functional features (biological functions, physiological functions, or cellular functions), organelle, cell type, tissue type, organ, development stage, or a combination of the foregoing. Examples of biological systems include, but are not limited to, the pulmonary, integument, skeletal, muscular, nervous (central and peripheral), endocrine, cardiovascular, immune, circulatory, respiratory, urinary, renal, gastrointestinal, colorectal, hepatic and reproductive systems. Other examples of biological systems include, but are not limited to, the various cellular functions in epithelial cells, nerve cells, blood cells, connective tissue cells, smooth muscle cells, skeletal muscle cells, fat cells, ovum cells, sperm cells, stem cells, lung cells, brain cells, cardiac cells, laryngeal cells, pharyngeal cells, esophageal cells, stomach cells, kidney cells, liver cells, breast cells, prostate cells, pancreatic cells, islet cells, testes cells, bladder cells, cervical cells, uterus cells, colon cells, and rectum cells. Some of the cells may be cells of cell lines, cultured in vitro or maintained in vitro indefinitely under appropriate culture conditions. Examples of cellular functions include, but are not limited to, cell proliferation (e.g., cell division), degeneration, regeneration, senescence, control of cellular activity by the nucleus, cell-to-cell signaling, cell differentiation, cell de-differentiation, secretion, migration, phagocytosis, repair, apoptosis, and developmental programming. Examples of cellular components that can be considered as biological systems include, but are not limited to, the cytoplasm, cytoskeleton, membrane, ribosomes, mitochondria, nucleus, endoplasmic reticulum (ER), Golgi apparatus, lysosomes, DNA, RNA, proteins, peptides, and antibodies.

A change or perturbation in a biological system relating to a phenotype of interest can be caused by a disease or it can caused by one or more agents over a period of time through exposure or contact with one or more parts of the biological system. An agent can be a single substance or a mixture of substances, including a mixture in which not all constituents are identified or characterized. The chemical and physical properties of an agent or its constituents may not be fully characterized. One or more agent can be the cause of a disease. An agent can be defined by its structure, its constituents, or a source that under certain conditions produces the agent. An example of an agent is a heterogeneous substance, that is a molecule or an entity that is not present in or derived from the biological system, and any intermediates or metabolites produced therefrom after contacting the biological system. An agent can be a carbohydrate, protein, lipid, nucleic acid, alkaloid, vitamin, metal, heavy metal, mineral, oxygen, ion, enzyme, hormone, neurotransmitter, inorganic chemical compound, organic chemical compound, environmental agent, microorganism, particle, environmental condition, environmental force, or physical force. Non-limiting examples of agents include but are not limited to nutrients, metabolic wastes, poisons, narcotics, toxins, therapeutic compounds, stimulants, relaxants, natural products, manufactured products, food substances, pathogens (prion, virus, bacteria, fungi, protozoa), particles or entities whose dimensions are in or below the micrometer range, by-products of the foregoing and mixtures of the foregoing. Non-limiting examples of a physical agent include radiation, electromagnetic waves (including sunlight), increase or decrease in temperature, shear force, fluid pressure, electrical discharge(s) or a sequence thereof, or trauma.

Non-limiting examples of an agent relating to a consumer product may include aerosol generated by heating tobacco, aerosol generated by combusting tobacco, tobacco smoke, cigarette smoke, and any of the gaseous constituents or particulate constituents thereof. A perturbation can also be caused by withholding an agent (as described above) from or limiting supply of an agent to one or more parts of a biological system. For example, a perturbation can be caused by a decreased supply of or a lack of nutrients, water, carbohydrates, proteins, lipids, alkaloids, vitamins, minerals, oxygen, ions, an enzyme, a hormone, a neurotransmitter, an antibody, a cytokine, light, or by restricting movement of certain parts of an organism, or by constraining or requiring exercise.

In various implementations, high-throughput system-wide measurements for gene expression, protein expression or turnover, microRNA expression or turnover, post-translational modifications, protein modifications, translocations, antibody production metabolite profiles, or a combination of two or more of the foregoing are generated under various conditions including the respective controls. Functional outcome measurements are desirable in the methods described herein as they can generally serve as anchors for the assessment and represent clear steps in a disease etiology.

A "sample," as the term is used herein, refers to any biological sample that is isolated from a subject or an experimental system (*e.g.*, cell, tissue, organ, or whole animal), including clinical data and epidemiology data. A sample can include, without limitation, a single cell or multiple cells, cellular fraction, tissue biopsy, resected tissue, tissue extract, tissue, tissue culture extract, tissue culture medium, exhaled gases, whole blood, platelets, serum, plasma, erythrocytes, leucocytes, lymphocytes, neutrophils, macrophages, B cells or a subset thereof, T cells or a subset thereof, a subset of hematopoietic cells, endothelial cells, synovial fluid, lymphatic fluid, ascites fluid, interstitial fluid, bone marrow, cerebrospinal fluid, pleural effusions, tumor infiltrates, saliva, mucous, sputum, semen, sweat, urine, or any other bodily fluids. Samples can be obtained from a subject by means including but not limited to venipuncture, excretion, biopsy, needle aspirate, lavage, scraping, surgical resection, or other means known in the art.

During operation, for a given biological mechanism, an outcome, a perturbation, a disease or its symptoms, or a combination of the foregoing, the system 100 can generate a network perturbation amplitude (NPA) value, which is a quantitative measure of changes in the status of biological entities in a network.

The system 100 (FIG. 1) comprises one or more computerized network model(s) that are relevant to the health condition, disease, or biological outcome, of interest. One or more of these network models are based on prior biological knowledge and can be uploaded from an external source and curated within the system 100. The models can also be generated *de novo* within the system 100 based on measurements. Measurable elements are causally integrated into biological network models through the use of prior knowledge. Described below are the types of data that represent changes in a biological system of interest that can be used to generate or refine a network model, or that represent a response to a perturbation.

Referring to FIG. 2, at step 210, the systems response profile (SRP) engine 110 receives biological data. The SRP engine 110 may receive this data from a variety of different sources, and the data itself may be of a variety of different types. The biological data used by the SRP engine 110 may be drawn from the literature, databases (including data from preclinical, clinical and post-clinical trials of pharmaceutical products or medical devices), genome databases (genomic sequences and expression data, *e.g.,* Gene Expression Omnibus by National Center for Biotechnology Information or ArrayExpress by European Bioinformatics Institute (Parkinson et al. 2010, Nucl. Acids Res., doi: 10.1093/nar/gkq1040. Pubmed ID 21071405)), commercially available databases (e.g., Gene Logic, Gaithersburg, MD, USA) or experimental work. The data may include raw data from one or more different sources, such as *in vitro, ex vivo* or *in vivo* experiments using one or more species that are specifically designed for studying the effect of particular treatment conditions or exposure to particular agents. In vitro experimental systems may include tissue cultures or organotypical cultures (three-dimensional cultures) that represent key aspects of human disease. In such implementations, the agent dosage and exposure regimens for these experiments may substantially reflect the range and circumstances of exposures that may be anticipated for humans during normal use or activity conditions, or during special use or activity conditions. Experimental parameters and test conditions may be selected as desired to reflect the nature of the agent and the exposure conditions, molecules and pathways of the biological system in question, cell types and tissues involved, the outcome of interest, and aspects of disease etiology. Particular animal-model-derived molecules, cells or tissues may be matched with particular human molecule, cell or tissue cultures to improve translatability of animal-based findings.

The data received by SRP engine 110 many of which are generated by high-throughput experimental techniques, include but are not limited to that relating to nucleic acid (e.g., absolute or relative quantities of specific DNA or RNA species, changes in DNA sequence, RNA sequence, changes in tertiary structure, or methylation pattern as determined by sequencing, hybridization - particularly to nucleic acids on microarray, quantitative polymerase chain reaction, or other techniques known in the art), protein/peptide (*e.g.*, absolute or relative quantities of protein, specific fragments of a protein, peptides, changes in secondary or tertiary structure, or posttranslational modifications as determined by methods known in the art) and functional activities (e.g., catalytic activities, enzymatic activities, proteolytic activities, transcriptional regulatory activities, transport activities, binding affinities to certain binding partners) under certain conditions, among others. Modifications including posttranslational modifications of protein or peptide can include, but are not limited to, methylation, acetylation, farnesylation, biotinylation, stearoylation, formylation, myristoylation, palmitoylation, geranylgeranylation, pegylation, phosphorylation, sulphation, glycosylation, sugar modification, lipidation, lipid modification, ubiquitination, sumolation, disulphide bonding, cysteinylation, oxidation, glutathionylation, carboxylation, glucuronidation, and deamidation. In addition, a protein can be modified posttranslationally by a series of reactions such as Amadori reactions, Schiff base reactions, and Maillard reactions resulting in glycated protein products.

The data may also include measured functional outcomes, such as but not limited to those at a cellular level including cell proliferation, developmental fate, and cell death, at a physiological level, lung capacity, blood pressure, exercise proficiency. The data may also include a measure of disease activity or severity, such as but not limited to tumor metastasis, tumor remission, loss of a function, and life expectancy at a certain stage of disease. Disease activity can be measured by a clinical assessment the result of which is a value, or a set of values that can be obtained from evaluation of a sample (or population of samples) from a subject or subjects under defined conditions. A clinical assessment can also be based on the responses provided by a subject to an interview or a questionnaire.

This data may have been generated expressly for use in determining a systems response profile, or may have been produced in previous experiments or studies, or published in the literature. Generally, the data includes information relating to a molecule, biological structure, physiological condition, genetic trait, or phenotype. In some implementations, the data includes a description of the condition, location, amount, activity, or substructure of a molecule, biological structure, physiological condition, genetic trait, or phenotype. As will be described later, in a clinical setting, the data may include raw or processed data obtained from assays performed on samples obtained from human subjects or observations on the human subjects, exposed to an agent.

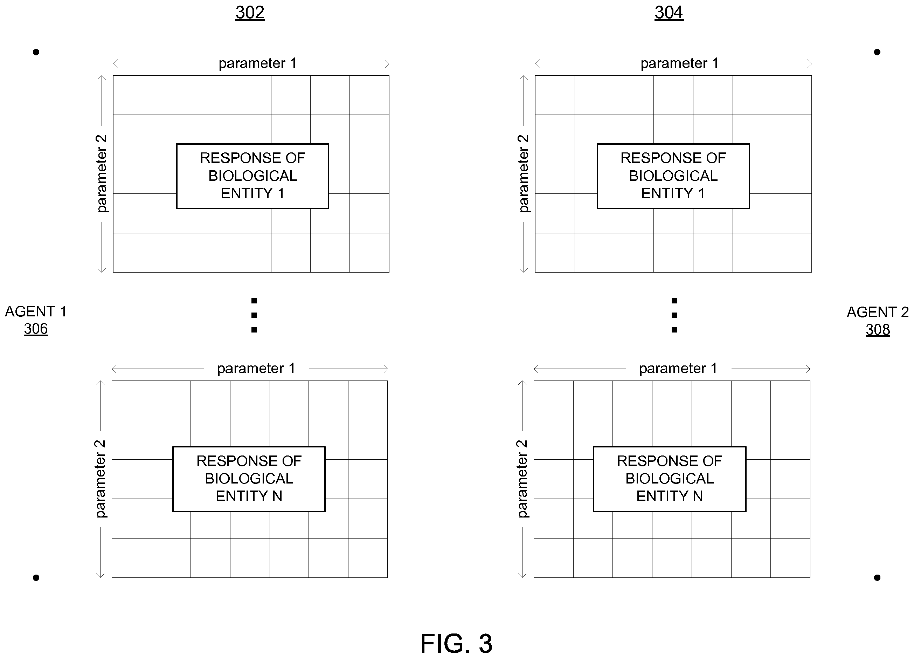

At step 212, the systems response profile (SRP) engine 110 generates systems response profiles (SRPs) based on the biological data received at step 212. This step may include one or more of background correction, normalization, fold-change calculation, significance determination and optionally, identification of a differential response (e.g., differentially expressed genes). However, this step may be performed without requiring a cutoff threshold. SRPs are representations that express the degree to which one or more measured entities within a biological system (e.g., a molecule, a nucleic acid, a peptide, a protein, a cell, etc.) are individually changed in response to a perturbation applied to the biological system (e.g., an exposure to an agent, pathological changes associated with the onset or progression of a disease). In one example, to generate an SRP, the SRP engine 110 collects a set of measurements for a given set of parameters (e.g., treatment or perturbation conditions) applied to a given experimental system (a "system-treatment" pair). FIG. 3 illustrates two SRPs: SRP 302 that includes biological activity data for N different biological entities undergoing a first treatment 306 with varying parameters (e.g., dose and time of exposure to a first treatment agent), and an analogous SRP 304 that includes biological activity data for the N different biological entities undergoing a second treatment 308. The data included in an SRP may be raw experimental data, processed experimental data (e.g., filtered to remove outliers, marked with confidence estimates, averaged over a number of trials), data generated by a computational biological model, or data taken from the scientific literature. An SRP may represent data in any number of ways, such as an absolute value, an absolute change, a fold-change, a logarithmic change, a function, and a table. The SRP engine 110 passes the SRPs to the network modeling engine 112.

While the SRPs derived in the previous step represent the experimental data from which the magnitude of network perturbation will be determined, it is the biological network models that are the substrate for computation and analysis. This analysis requires development of a detailed network model of the mechanisms and pathways relevant to a feature of the biological system. Such a framework provides a layer of mechanistic understanding beyond examination of gene lists that have been used in more classical gene expression analysis. A network model of a biological system is a mathematical construct that is representative of a dynamic biological system and that is built by assembling quantitative information about various basic properties of the biological system.

Returning to FIG. 2, at step 214, the network modeling engine 112 uses the systems response profiles (SRPs) from the SRP engine 110 with a network model based on the mechanism(s) or pathway(s) underlying a feature of a biological system of interest. In certain aspects, the network modeling engine 112 is used to identify networks already generated based on SRPs. The network modeling engine 112 may include components for receiving updates and changes to models. The network modeling engine 112 may also iterate the process of network generation, incorporating new data and generating additional or refined network models. The network modeling engine 112 may also facilitate the merging of one or more datasets or the merging of one or more networks. The set of networks drawn from a database may be manually supplemented by additional nodes, edges, or entirely new networks (e.g., by mining the text of literature for description of additional genes directly regulated by a particular biological entity). These networks contain features that may enable process scoring. Network topology is maintained; networks of causal relationships can be traced from any point in the network to a measurable entity. Further, the models are dynamic and the assumptions used to build them can be modified or restated and enable adaptability to different tissue contexts and species. This allows for iterative testing and improvement as new knowledge becomes available. The network modeling engine 112 may remove nodes or edges that have low confidence or which are the subject of conflicting experimental results in the scientific literature. The network modeling engine 112 may also include additional nodes or edges that may be inferred using supervised or unsupervised learning methods (e.g., metric learning, matrix completion, pattern recognition).

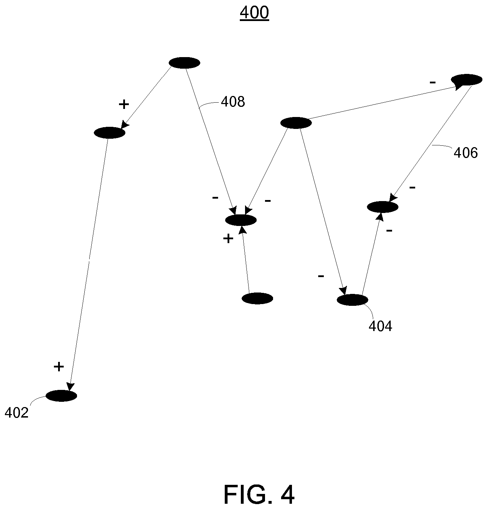

In certain aspects, a biological system is modeled as a mathematical graph consisting of vertices (or nodes) and edges that connect the nodes. For example, FIG. 4 illustrates a simple network 400 with 9 nodes (including nodes 402 and 404) and edges (406 and 408). The nodes can represent biological entities within a biological system, such as, but not limited to, compounds, DNA, RNA, proteins, peptides, antibodies, cells, tissues, and organs. The edges can represent relationships between the nodes. The edges in the graph can represent various relations between the nodes. For example, edges may represent a "binds to" relation, an "is expressed in" relation, an "are co-regulated based on expression profiling" relation, an "inhibits" relation, a "co-occur in a manuscript" relation, or "share structural element" relation. Generally, these types of relationships describe a relationship between a pair of nodes. The nodes in the graph can also represent relationships between nodes. Thus, it is possible to represent relationships between relationships, or relationships between a relationship and another type of biological entity represented in the graph. For example a relationship between two nodes that represent chemicals may represent a reaction. This reaction may be a node in a relationship between the reaction and a chemical that inhibits the reaction.

A graph may be undirected, meaning that there is no distinction between the two vertices associated with each edge. Alternatively, the edges of a graph may be directed from one vertex to another. For example, in a biological context, transcriptional regulatory networks and metabolic networks may be modeled as a directed graph. In a graph model of a transcriptional regulatory network, nodes would represent genes with edges denoting the regulatory relationships between them. An edge of a graph may also include a sign indicating whether the value represented by a node connected to the edge increases or decreases in association with or as a result of a change in another node connected to the edge. As another example, protein-protein interaction networks describe direct physical interactions between the proteins in an organism's proteome and there is often no direction associated with the interactions in such networks. Thus, these networks may be modeled as undirected graphs. Certain networks may have both directed and undirected edges. The entities and relationships (i.e., the nodes and edges) that make up a graph may be stored as a web of interrelated nodes in a database in system 100.

The knowledge represented within the database may be of various different types, drawn from various different sources. For example, certain data may represent a genomic database, including information on genes, and relations between them. In such an example, a node may represent an oncogene, while another node connected to the oncogene node may represent a gene that inhibits the oncogene. The data may represent proteins, and relations between them, diseases and their interrelations, and various disease states. There are many different types of data that can be combined in a graphical representation. The computational models may represent a web of relations between nodes representing knowledge in, e.g., a DNA dataset, an RNA dataset, a protein dataset, an antibody dataset, a cell dataset, a tissue dataset, an organ dataset, a medical dataset, an epidemiology dataset, a chemistry dataset, a toxicology dataset, a patient dataset, and a population dataset. As used herein, a dataset is a collection of numerical values resulting from evaluation of a sample (or a group of samples) under defined conditions. Data sets can be obtained, for example, by experimentally measuring quantifiable entities of the sample; or alternatively, or from a service provider such as a laboratory, a clinical research organization, or from a public or proprietary database. Datasets may contain data and biological entities represented by nodes, and the nodes in each of the datasets may be related to other nodes in the same dataset, or in other datasets. Moreover, the network modeling engine 112 may generate computational models that represent genetic information, in, e.g., DNA, RNA, protein or antibody dataset, to medical information, in medical dataset, to information on individual patients in patient dataset, and on entire populations, in epidemiology dataset. In addition to the various datasets described above, there may be many other datasets, or types of biological information that may be included when generating a computation model. For example, a database could further include medical record data, structure/activity relationship data, information on infectious pathology, information on clinical trials, exposure pattern data, data relating to the history of use of a product, and any other type of life science-related information.

The network modeling engine 112 may generate one or more network models representing, for example, the regulatory interaction between genes, interaction between proteins or complex bio-chemical interactions within a cell or tissue. The network models generated by the network modeling engine 112 may include static and dynamic models. The network modeling engine 112 may employ any applicable mathematical schemes to represent the system, such as hyper-graphs and weighted bipartite graphs, in which two types of nodes are used to represent reactions and compounds. The network modeling engine 112 may also use other inference techniques to generate network models, such as an analysis based on over-representation of functionally-related genes within the differentially expressed genes, Bayesian network analysis, a graphical Gaussian model technique or a gene relevance network technique, to identify a relevant biological network based on a set of experimental data (e.g., gene expression, metabolite concentrations, cell response, etc.).

As described above, the network model is based on mechanisms and pathways that underlie the functional features of a biological system. The network modeling engine 112 may generate or contain a model representative of an outcome regarding a feature of the biological system that is relevant to the onset and progression of a disease or the study of the long-term health risks or health benefits of agents. Accordingly, the network modeling engine 112 may generate or contain a network model for various mechanisms of cellular function, particularly those that relate or contribute to a feature of interest in the biological system, including but not limited to cellular proliferation, cellular stress, cellular regeneration, apoptosis, DNA damage/repair or inflammatory response. In other embodiments, the network modeling engine 112 may contain or generate computational models that are relevant to acute systemic toxicity, carcinogenicity, dermal penetration, cardiovascular disease, pulmonary disease, ecotoxicity, eye irrigation/corrosion, genotoxicity, immunotoxicity, neurotoxicity, pharmacokinetics, drug metabolism, organ toxicity, reproductive and developmental toxicity, skin irritation/corrosion or skin sensitization. Generally, the network modeling engine 112 may contain or generate computational models for status of nucleic acids (DNA, RNA, SNP, siRNA, miRNA, RNAi), proteins, peptides, antibodies, cells, tissues, organs, and any other biological entity, and their respective interactions. In one example, computational network models can be used to represent the status of the immune system and the functioning of various types of white blood cells during an immune response or an inflammatory reaction. In other examples, computational network models could be used to represent the performance of the cardiovascular system and the functioning and metabolism of endothelial cells.

In some implementations of the present disclosure, the network is drawn from a database of causal biological knowledge. This database may be generated by performing experimental studies of different biological mechanisms to extract relationships between mechanisms (e.g., activation or inhibition relationships), some of which may be causal relationships, and may be combined with a commercially-available database such as the Genstruct Technology Platform or the Selventa Knowledgebase, curated by Selventa Inc. of Cambridge, Massachusetts, USA. Using a database of causal biological knowledge, the network modeling engine 112 may identify a network that links the perturbations 102 and the measurables 104. In certain implementations, the network modeling engine 112 extracts causal relationships between biological entities using the systems response profiles from the SRP engine 110 and networks previously generated in the literature. The database may be further processed to remove logical inconsistencies and generate new biological knowledge by applying homologous reasoning between different sets of biological entities, among other processing steps. As used herein, the term "causal biological network model" refers to a collection of biological entities ("nodes") and the relationships between those entities ("edges") which represent specific types of cause-and-effect relationships.

In certain implementations, the network model extracted from the database is based on reverse causal reasoning (RCR), an automated reasoning technique that processes networks of causal relationships to formulate mechanism hypotheses. The network modeling engine then evaluates those mechanism hypotheses against datasets of differential measurements. Each mechanism hypothesis links a biological entity to measurable quantities that it can influence. For example, measurable quantities can include an increase or decrease in concentration, number or relative abundance of a biological entity, activation or inhibition of a biological entity, or changes in the structure, function or logical of a biological entity, among others. RCR uses a directed network of experimentally-observed causal interactions between biological entities as a substrate for computation. The directed network may be expressed in Biological Expression Language™ (BEL™), a syntax for recording the inter-relationships between biological entities. The RCR computation specifies certain constraints for network model generation, such as but not limited to path length (the maximum number of edges connecting an upstream node and downstream nodes), and possible causal paths that connect the upstream node to downstream nodes. The output of RCR is a set of mechanism hypotheses that represent upstream controllers of the differences in experimental measurements, ranked by statistics that evaluate relevance and accuracy. The mechanism hypotheses output can be assembled into causal chains and larger networks to interpret the dataset at a higher level of interconnected mechanisms and pathways.

One type of mechanism hypothesis comprises a set of causal relationships that exist between a node representing a potential cause (the upstream node or controller) and nodes representing the measured quantities (the downstream nodes). This type of mechanism hypothesis can be used to make predictions, such as if the abundance of an entity represented by an upstream node increases, the downstream nodes linked by causal increase relationships would be inferred to increase, and the downstream nodes linked by causal decrease relationships would be inferred to decrease.

A mechanism hypothesis can represent the relationships between a set of measured data, for example, gene expression data, and a biological entity that is a known controller of those genes. Additionally, these relationships include the sign (positive or negative) of influence between the upstream entity and the differential expression of the downstream entities (for example, downstream genes). The downstream entities of a mechanism hypothesis can be drawn from a database of literature-curated causal biological knowledge. In certain implementations, the causal relationships of a mechanism hypothesis that link the upstream entity to downstream entities, in the form of a computable causal network model, are the substrate for the calculation of network changes by the NPA scoring methods.

In certain embodiments, a complex causal network model of biological entities can be transformed into a single causal network model by collecting the individual mechanism hypothesis representing various features of the biological system in the model and regrouping the connections of all the downstream entities (e.g., downstream genes) to a single upstream entity or process, thereby representing the whole complex causal network model; this in essence is a flattening of the underlying graph structure. Changes in the features and entities of a biological system as represented in a network model can thus be assessed by combining individual mechanism hypotheses.

In certain implementations, the system 100 may contain or generate a computerized model for the mechanism of cell proliferation when the cells have been exposed to cigarette smoke. In such an example, the system 100 may also contain or generate one or more network models representative of the various health conditions relevant to cigarette smoke exposure, including but not limited to cancer, pulmonary diseases and cardiovascular diseases. In certain aspects, these network models are based on at least one of the perturbations applied (e.g., exposure to an agent), the responses under various conditions, the measureable quantities of interest, the outcome being studied (e.g., cell proliferation, cellular stress, inflammation, DNA repair), experimental data, clinical data, epidemiological data, and literature.

As an illustrative example, the network modeling engine 112 may be configured for generating a network model of cellular stress. The network modeling engine 112 may receive networks describing relevant mechanisms involved in the stress response known from literature databases. The network modeling engine 112 may select one or more networks based on the biological mechanisms known to operate in response to stresses in pulmonary and cardiovascular contexts. In certain implementations, the network modeling engine 112 identifies one or more functional units within a biological system and builds a larger network model by combining smaller networks based on their functionality. In particular, for a cellular stress model, the network modeling engine 112 may consider functional units relating to responses to oxidative, genotoxic, hypoxic, osmotic, xenobiotic, and shear stresses. Therefore, the network components for a cellular stress model may include xenobiotic metabolism response, genotoxic stress, endothelial shear stress, hypoxic response, osmotic stress and oxidative stress. The network modeling engine 112 may also receive content from computational analysis of publicly available transcriptomic data from stress relevant experiments performed in a particular group of cells.

When generating a network model of a biological mechanism, the network modeling engine 112 may include one or more rules. Such rules may include rules for selecting network content, types of nodes, and the like. The network modeling engine 112 may select one or more data sets from experimental data database 106, including a combination of *in vitro* and *in vivo* experimental results. The network modeling engine 112 may utilize the experimental data to verify nodes and edges identified in the literature. In the example of modeling cellular stress, the network modeling engine 112 may select data sets for experiments based on how well the experiment represented physiologically-relevant stress in non-diseased lung or cardiovascular tissue. The selection of data sets may be based on the availability of phenotypic stress endpoint data, the statistical rigor of the gene expression profiling experiments, and the relevance of the experimental context to normal non-diseased lung or cardiovascular biology, for example.

After identifying a collection of relevant networks, the network modeling engine 112 may further process and refine those networks. For example, in some implementations, multiple biological entities and their connections may be grouped and represented by a new node or nodes (e.g., using clustering or other techniques).

The network modeling engine 112 may further include descriptive information regarding the nodes and edges in the identified networks. As discussed above, a node may be described by its associated biological entity, an indication of whether or not the associated biological entity is a measurable quantity, or any other descriptor of the biological entity. An edge may be described by the type of relationship it represents (e.g., a causal relationship such as an up-regulation or a down-regulation, a correlation, a conditional dependence or independence), the strength of that relationship, or a statistical confidence in that relationship, for example. In some implementations, for each treatment, each node that represents a measureable entity is associated with an expected direction of activity change (*i.e.,* an increase or decrease) in response to the treatment. For example, when a bronchial epithelial cell is exposed to an agent such as tumor necrosis factor (TNF), the activity of a particular gene may increase. This increase may arise because of a direct regulatory relationship known from the literature (and represented in one of the networks identified by network modeling engine 112) or by tracing a number of regulation relationships (e.g., autocrine signaling) through edges of one or more of the networks identified by network modeling engine 112. In some implementations, an edge between first and second nodes in a network is associated with a signed value that represents how an increase in the entity associated with the first node may affect the entity associated with a second node. As shown in FIG. 4, these signed values may take the form of "+" and "-" signs, representing activation and suppression, respectively. In some cases, the network modeling engine 112 may identify an expected direction of change, in response to a particular perturbation, for each of the measureable entities. When different pathways in the network indicate contradictory expected directions of change for a particular entity, the two pathways may be examined in more detail to determine the net direction of change, or measurements of that particular entity may be discarded.

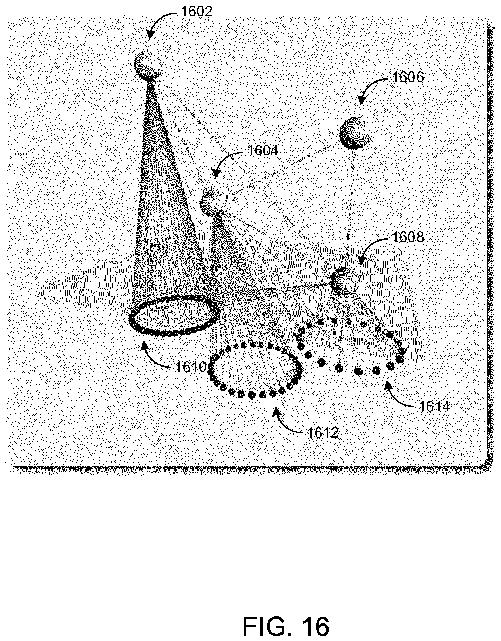

In some implementations, a subset of the nodes in a network (referred to herein as "backbone nodes") represent biological processes or key actors in a biological process in a causal biological network model that are not measured, and a subset of the nodes in a network (referred to herein as "supporting nodes") represent measurable entities, such as gene expression levels. FIG. 16 depicts an exemplary network that includes four backbone nodes 1602, 1604, 1606 and 1608 and edges between the backbone nodes and from the backbone nodes to groups of supporting gene expression nodes 1610, 1612 and 1614. Each edge in FIG. 16 is directed (*i.e.,* representing the direction of a cause-and-effect relationship) and signed (*i.e.,* representing positive or negative regulation). These networks may represent a set of causal relationships that connect particular biological entities (e.g., from something as specific as the increase in abundance or activation of a particular kinase to something as complex as a growth factor signaling pathway) to the measurable downstream entities (e.g., gene expression values) that are positively or negatively regulated by these biological entities. Without being bound by any theory, using measured downstream effects to infer the activity of upstream entities may be advantageous as compared to "forward" inferences (e.g., that mRNA expression changes are always directly correlated with protein activity changes) because these forward inferences may not take into account the effects of translational or post-translational regulation on protein activity.

Construction of such a network may be an iterative process. Delineation of boundaries of the network may be guided by literature investigation of mechanisms and pathways relevant to the process of interest (e.g., cell proliferation in the lung). Causal relationships describing these pathways may be extracted from prior knowledge to nucleate a network. The literature-based network may be verified using high-throughput data sets that contain the relevant phenotypic endpoints. SRP engine 110 can be used to analyze the data sets, the results of which can be used to confirm, refine, or generate network models.

In some implementations, the building of a causal biological network model utilized by the computational systems described herein may proceed according to the following multi-step iterative process. First, a team of scientists defines the biological boundaries of the network using a survey of relevant scientific literature into the signaling pathways relevant to the process of interest (e.g., cell proliferation in the lung) and inputs these boundaries to the network modeling engine 112. Cause-and-effect relationships describing these pathways are extracted from the research literature and from databases such as Selventa's Knowledgebase, a unified collection of over 1.5 million cause-and-effect biological relationships. Nodes in the networks may include biological entities (such as protein abundances, and protein activities) and biological processes (e.g., apoptosis). Edges are relationships between the nodes, and represent directional cause-and-effect relationships between the entities (e.g., the transcriptional activity of NFKB directly causes an increase in the gene expression of BCL2). Some edges connect different forms of a biological entity, such as the protein abundance to its phosphorylated form (e.g., TP53 protein abundance to TP53 phosphorylated at serine 15). The resulting network represents the biology underneath the cellular process of interest. Second, the network modeling engine 112 subjects molecular profiling data to computational deconvolution using Reverse Causal Reasoning. As described elsewhere herein, RCR is a computational technique that receives gene expression profiling data as an input and generates predicted values for the activity states of biological entities (i.e., nodes in the network) according to statistical and biological criteria. Hypothesized upstream controllers of the observed experimental data are drawn from those computational predictions. Some specific types of edges can describe causal relationships between an upstream biological activity and any type of high-throughput data. In the case of transcriptomic data, causal relationships between a given entity or process and the high throughput gene expression data may identify a causal "gene expression signature" for the given entity or process (for example, the activity of a particular kinase), as discussed in detail below. Third, the network modeling engine 112 submits the content and connectivity of the causal biological network model to a terminal round of manual review by discipline-specific scientific experts. Ultimately, this three-step methodology may result in a computationally advantageous network model whose edges are supported by published literature and the scientific community.

In some aspects, the computational methods and systems provided herein calculate NPA scores based on experimental data and computational network models. The computational network models may be generated by the system 100, imported into the system 100, or identified within the system 100 (e.g., from a database of biological knowledge). Experimental measurements that are identified as downstream effects of a perturbation within a network model are combined in the generation of a network-specific response score. Accordingly, at step 216, the network scoring engine 114 generates NPA scores for each perturbation using the networks identified at step 214 by the network modeling engine 112 and the SRPs generated at step 212 by the SRP engine 110. An NPA score quantifies a biological response to a treatment (represented by the SRPs) in the context of the underlying relationships between the biological entities (represented by the identified networks). The network scoring engine 114 may include hardware and software components for generating NPA scores for each of the networks contained in or identified by the network modeling engine 112.

The network scoring engine 114 may be configured to implement any of a number of scoring techniques, including techniques that generate scalar- or vector-valued scores indicative of the magnitude and topological distribution of the response of the network to the perturbation. A number of scoring techniques are now described.

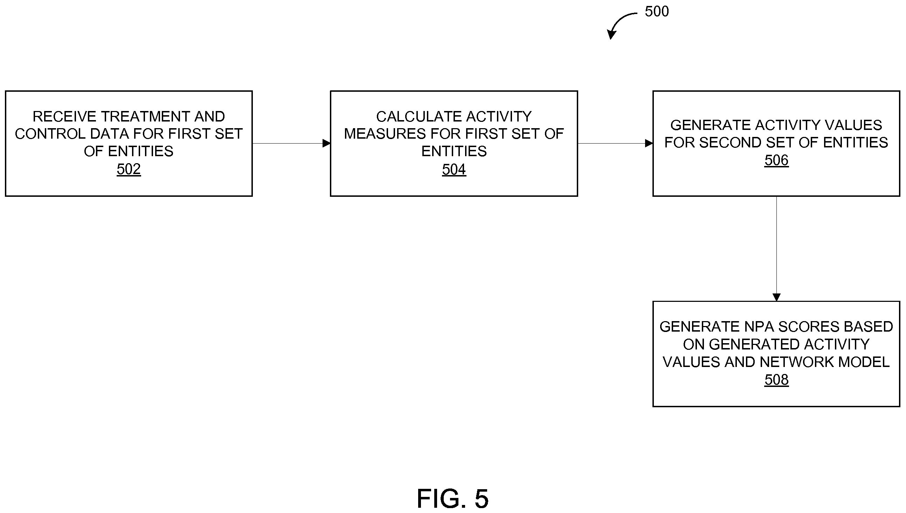

FIG. 5 is a flow diagram of an illustrative process 500 for quantifying the perturbation of a biological system in response to an agent. The process 500 may be implemented by the network scoring engine 114 or any other suitably configured component or components of the system 100, for example. At the step 502, the network scoring engine 114 receives treatment and control data for a first set of biological entities in a biological system (referred to as the "supporting entities"). The treatment data corresponds to a response of the supporting entities to an agent, while the control data corresponds to the response of the supporting entities to the absence of the agent. The biological system includes the supporting entities (for which treatment and control data is received at the step 502), as well as a second set of biological entities for which no treatment and control data may be received (referred to as the "backbone entities"). Each biological entity in the biological system interacts with at least one other of the biological entities in the biological system, and in particular, at least one supporting entity interacts with at least one backbone entity. The relationship between biological entities in the biological system may be represented by a computational network model that includes a first set of nodes representing the supporting entities, a second set of nodes representing the backbone entities, and edges that connect the nodes and represent relationships between the biological entities. The computational network model may also include directions values (also referred to as a sign) for the nodes, which represent the expected direction of change between the control and treatment data (e.g., activation or suppression). Examples of such network models are described in detail above.

At the step 504, the network scoring engine 114 calculates activity measures for the supporting entities. Each activity measure represents a difference between the treatment data and the control data for a particular supporting entity. Because of the correspondence between the supporting entities and the first set of nodes in the computational network model, the step 504 also calculates activity measures for the first set of nodes in the computational network model. In some implementations, the activity measures may include a fold-change. The fold-change may be a number describing how much a node measurement changes going from an initial value to a final value between control data and treatment data, or between two sets of data representing different treatment conditions. The fold-change number may represent the logarithm of the fold-change of the activity of the biological entity between the two conditions. The activity measure for each node may include a logarithm of the difference between the treatment data and the control data for the biological entity represented by the respective node. In certain implementations, the computerized method includes generating, with a processor, a confidence interval for each of the generated scores.

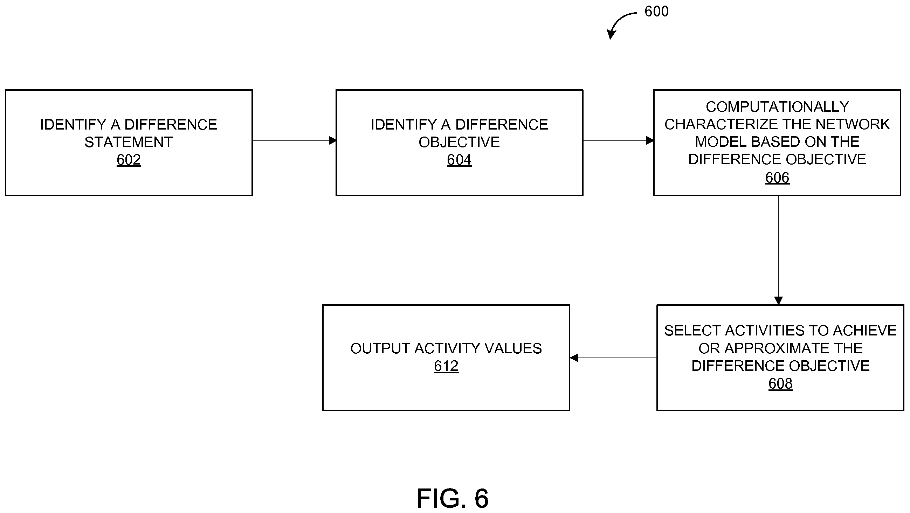

At the step 506, the network scoring engine 114 generates activity values for the backbone entities. Because no treatment and control data were received for the backbone entities here, the activity values generated at the step 506 represent inferred activity values, and are based on the first set of activity measures and the computational network model. The activity values inferred for the backbone entities (corresponding to a second set of nodes in the computational network model) may be generated according to any of a number of inference techniques; several implementations are described below with reference to FIG. 6. The activity values generated for backbone entities at the step 506 illuminate the behavior of biological entities that are not measured directly, using the relationships between entities provided by the network model.

At the step 508, the network scoring engine 114 calculates an NPA score based on the activity values generated at the step 506. The NPA score represents the perturbation of the biological system to the agent (as reflected in the difference between the control and treatment data), and is based on the activity values generated at the step 506 and the computational network model. In some implementations, the NPA score calculated at the step 508 may be calculated in accordance with

$$NPA(\mathcal{G}, \beta) = \frac{1}{|\{x \to y\} \text{ s.t. } x,y \notin V_0|} \sum_{\substack{x \to y \\ \text{s.t } x,y \notin V_0}} (f(x) + sign(x \to y)f(y))^2 \tag{1}$$

where $V_o$ denotes the set of supporting entities (*i.e.*, those for which treatment and control data are received at the step 502), $f(x)$ denotes the activity value generated at the step 508 for the biological entity x, and *sign (x→y)* denotes the direction value of the edge in the computational network model that connects the node representing biological entity x to the node representing biological entity *y*. If the vector of activity values associated with the set of backbone entities is denoted $f_2$, the network scoring engine 114 can be configured to calculate the NPA score via the quadratic form

$$NPA = f_2^T Q f_2 \tag{2}$$

where

$$Q = (diag(out|_{l^2(V \setminus V_0)}) + diag(in|_{l^2(V \setminus V_0)}) - (-A - A^T))|_{l^2(V \setminus V_0)} \in l^2(V \setminus V_0) \tag{3}$$

*diag(out)* denotes the diagonal matrix with the out-degree of each node in the second set of nodes, *diag(in)* denotes the diagonal matrix with the in-degree of each node in the second set of nodes, *V* is the set of all nodes in the network, and A denotes the adjacency matrix of the computational network model limited to only nodes representing backbone entities and defined in accordance with

$$A_{xy} = \begin{cases} sign(x \to y) & \text{if } x \to y \\ 0 & \text{else} \end{cases} \tag{4}$$

If *A* is a weighted adjacency matrix, then element (x,y) of A may be multiplied by a weight factor *w(x→y)*. In some scenarios, some backbone nodes may have more supporting gene expression evidence than other backbone nodes due to the so-called literature bias in which some entities are studied more than others. The result in the causal computation biological model is that nodes with more supporting evidence will have a higher degree then less "rich" nodes. When compounded with the possibility that a majority of the evidence have very low signal, the inferred node activity values might be systematically one of the nodes with the lowest value. To address this issue, in some implementations, the weights associated with an edge from a node to one of the node's *N* downstream nodes is set to *1/N*. This modification may advantageously emphasize the backbone structure (which captures important aspects of the biology) and balance the importance of the backbone and the supporting nodes within the causal biological network model computations.

The step 508 may also include calculating confidence intervals for the NPA score. In some implementations, the activity values $f_2$ are assumed to follow a multivariate normal distribution $N(\mu, \Sigma)$, then an NPA score calculated in accordance with Eq.2 will have an associated variance that may be calculated in accordance with

$$\mathrm{var}\left(f^{T}Qf\right)=2tr\left(Q\Sigma Q\Sigma\right)+4\mu^{T}Q\Sigma Q\mu \tag{5}$$

In some implementations, such as those that operate in accordance with Eq. 5, the NPA score has a quadratic dependence on the activity values. The network scoring engine 114 may be further configured to use the variance calculated in accordance with Eq. 5 to generate a conservative confidence interval by, among other methods, applying Chebyshev's inequality.

FIG. 6 is a flow diagram of an illustrative process 600 for generating activity values for a set of nodes. The process 600 may be performed at step 506 of the process 500 of FIG. 5, for example, and is described as being performed by the network scoring engine 114 for ease of illustration. At step 602, the network scoring engine 114 identifies a difference statement. A difference statement is an expression or other executable statement that represents the difference between the activity measure or value of a particular biological entity and the activity measure or value of biological entities to which the particular biological entity is connected. In the language of the computational network model representing the biological system of interest, a difference statement represents the difference between the activity measure or value of a particular node in the network model and the activity measure or value of nodes to which the particular node is connected via an edge. The difference statement may depend on any one or more of the nodes in the computational network model. In some embodiments, the difference statement depends on the activity values of each node in the second set of nodes discussed above with respect to the step 506 of FIG. 5 *(i.e.,* those nodes for which no treatment or control data is available, and whose activity values are inferred from treatment or control data associated with other nodes and the computational network model).

In some implementations, the network scoring engine 114 identifies the following difference statement at the step 602:

$$\sum_{x \to y}\left(f(x)-sign(x \to y)f(y)\right)^{2}w(x \to y) \tag{6}$$

where f(x) denotes an activity value (for nodes x representing backbone entities) or measure (for nodes x representing supporting entities), sign(x→y) denotes the direction value (or sign, representing activation or inhibition) of the edge in the computational network model that connects the node representing biological entity x to the node representing biological entity y, and w(x→y) denotes a weight associated with the edge connecting the nodes representing entities x and y. For ease of illustration, the remaining discussion will assume that w(x→y) is equal to one, but one of ordinary skill in the art will easily track non-unity weights through the discussion of the difference statement of Eq. 6 (*i.e.,* by using a weighted adjacency matrix as described above with reference to Eq. 5).

The network scoring engine 114 may implement the difference statement of Eq. 6 in many different ways, including any of the following equivalent statements:

$$
\begin{aligned}
& \sum_{x \to y}(f(x) - sign(x \to y)f(y))^{2} \\
={}& \sum_{x}\sum_{y:x \to y} f(x)^{2} + f(y)^{2} - 2sign(x \to y)f(x)f(y) \\
={}& \sum_{x} f(x)^{2} \cdot out(x) + \sum_{y} f(y)^{2} \cdot in(y) - 2\sum_{x \to y} sign(x \to y)f(x)f(y) \\
={}& f^{T}(diag(out) + diag(in))f - f^{T}(A + A^{T})f
\end{aligned}
\tag{7}
$$

At the step 604, the network scoring engine 114 identifies a difference objective. The difference objective represents an optimization goal for the value of the difference statement towards which the network scoring engine 114 will select the activity values for the backbone entities. The difference objective may specify that the difference statement is to be maximized, minimized, or made as close as possible to a target value. The difference objective may specify the biological entities for which activity values are to be chosen, and may establish constraints on the range of activity values that are allowed for each entity. In some implementations, the difference objective is to minimize the difference statement of Eq. 6 over all backbone entities discussed above with reference to the step 506 of FIG. 5, with the constraint that the activities of the supporting entities (*i.e.,* those for which treatment and control data is available) be equal to the activity measures calculated at the step 504 of FIG. 5. This difference objective may be written as the following computational optimization problem:

$$\operatorname{argmin}_{f \in l^2(V)} \sum_{x \to y} (f(x) - sign(x \to y)f(y))^2 \cdot w(x \to y) \text{ such that } f|_{V_0} = \beta \qquad \text{(8)}$$

where $\beta$ represents the activity measure calculated at the step 504 of FIG. 5 for each of the supporting entities. In some implementations, to accommodate differential data with a low signal-to-noise ratio, *(1 - Pvalue)* $\beta$ may be used instead of $\beta$ in Eq. 8. The variance of an NPA score calculated in accordance with this alternative for $\beta$ may be calculated as described in Martin et al., BMC Syst Biol. 2012 May 31; 6(1):54.

To address the difference objective identified at the step 604, the network scoring engine 114 is configured to proceed to the step 606 to computationally characterize the network model based on the difference objective. The computational network model representing the biological system may be characterized in any number of ways (e.g., via a weighted or non-weighted adjacency matrix *A* as discussed above). Different characterizations may be better suited to different difference objectives, improving the performance of the network scoring engine 114 in calculating NPA scores. For example, when the difference objective is formulated according to Eq. 8, above, the network scoring engine 114 may be configured to characterize the computational network model using a signed Laplacian matrix defined in accordance with

$$L = \left( diag(out) + diag(in) - \left( A + A^T \right) \right) \qquad \text{(9)}$$

Given this characterization, the difference objective of Eq. 8 can be represented as

$$\arg \min_{f \in l^2(V_0)} f^T L f \quad such\,that \quad f\big|_{V_0} = \beta \qquad \text{(10)}$$

The network scoring engine 114 may be configured to characterize the computation network model at a second level by partitioning the network model into four components: edges among the supporting nodes, edges from the supporting nodes to the backbone nodes, edges from the backbone nodes to the supporting nodes, and edges among the backbone nodes. Computationally, the network scoring engine 114 may implement this additional characterization by partitioning the Laplacian matrix into four sub-matrices (one for each of these components) and partitioning the vector of activities f into two sub-vectors (one for the activities of the supporting nodes and one for the activities of the backbone nodes). This recharacterization of the difference statement of Eq. 10 may be written as:

$$f^T \left( \begin{array}{c|c} L_1 & L_2 \\ \hline L_2^T & L_3 \end{array} \right) f = \left( \, f_1^T \mid f_2^T \, \right) \left( \begin{array}{c|c} L_1 & L_2 \\ \hline L_2^T & L_3 \end{array} \right) \left( \begin{array}{c} f_1 \\ \hline f_2 \end{array} \right) = f_1^T L_1 f_1 + f_1^T L_2 f_2 + f_2^T L_2^T f_1 + f_2^T L_3 f_2 \qquad \text{(11)}$$

At the step 606, the network scoring engine 114 selects activity values to achieve or approximate the difference objective. Many different computational optimization routines are known in the art, and may be applied to any difference objective identified at the step 604. In implementations in which the difference objective of Eq. 10 is identified at the step 604, the network scoring engine 114 may be configured to select the values of f2 that minimize the expression of Eq. 11 by taking a (numerical or analytical) derivative of Eq. 11 with respect to $f_2$, setting the derivative equal to zero, and rearranging to isolate an expression for $f_2$. Since

$$\frac{\partial}{\partial f_2} (f^T L f) = 2L_2^T f_1 + 2L_3 f_2 \qquad \text{(12)}$$

the network scoring engine 114 may be configured to calculate f2 in accordance with:

$$f_2 = -L_3^{-1} L_2^T f_1 \equiv K f_1 \tag{13}$$

In some implementations, if $L_3$ is singular, the Moore-Penrose generalized inverse is used. Since f1 is a vector of the calculated activity measures for the supporting entities (for which treatment and control data is available), the activity values for the backbone entities may be represented as a linear combination of the calculated activity measures in accordance with Eq. 13. As in Eq. 13, the activity values may depend on edges between nodes representing supporting entities and nodes representing backbone entities within the first computational network model, and may also depend on edges between nodes in the second set of nodes within the computational causal network model. In some implementations (such as those that operate in accordance with Eq. 13), the activity values do not depend on edges between nodes representing supporting entities within the computational network model.

At the step 608, the network scoring engine 114 provides the activity values generated at the step 606. In some implementations, the activity values are displayed for a user. In some implementations, the activity values are used at the step 508 of FIG. 5 to calculate an NPA score as described above. In some implementations, variance and confidence information for the activity values may also be generated at the step 608. For example, if the activity values and measures may be assumed to approximately follow a multivariate normal distribution, $N(\mu,\Sigma)$, then Kf will also follow a multivariate normal distribution with

$$\mathrm{var}\left(Kf\right) = K\Sigma K^T. \tag{14}$$

In this case, confidence intervals for the inferred activity values may be calculated using standard statistical techniques with $K = -L_3^{-1}L_2^T$ and $\Sigma = diag(var(\beta))$.

Since an NPA score may be computed as a quadratic form (as shown above), the network scoring engine 114 may generate a significant (with respect to the biological variability) score even though the input data do not reflect actual perturbation of the mechanisms in the model. In some implementations, the significance of an NPA or other score depends on whether the variability between biological samples is consistent at multiple levels of the NPA or other score calculation (e.g., fold-changes, backbone scores and NPA scores). To assess if a network is really perturbed (i.e., that the biology described in the model is reflected in the data), companion statistics may be used to help determine whether the extracted signal is specific to the network structure or is inherent within the collected data. Two permutation tests may be particularly useful in assessing whether the observed signal is more representative of a property inherent to the data or the structure given by the causal biological network model. The first test quantifies the importance of the position of the supporting nodes within the network to the measured signal. To do so, the gene labels are reshuffled, NPA scores are re-computed and a permutation P-value is derived. The second test quantifies the importance of the backbone network structure to the measured signal. In this test, the edges of the backbone model are randomly permuted, NPA scores are re-computed and a permutation P-value is derived. The latter test evaluates the importance of the cause-and-effect relationships encoded in the backbone of the network while the former test evaluates whether the measured signal is specific to the underlying evidences in the model. The network is considered to be "perturbed" if both P-values are low (in some implementations, 0.05 or less).

As noted above, the network scoring engine 114 may be configured to calculate confidence intervals for activity values and NPA scores. To do so, the network scoring engine 114 may compute the activity measures (denoted here as $\beta$) as described above with reference to step 504 of FIG. 5. In some implementations, the activity measures may be a fold-change value or a weighted fold-change value (weighted, e.g., using an associated false non-discovery rate) determined by the Limma R statistical analysis package or by another standard statistical technique. The network scoring engine 114 may compute the variances associated with the activity measures (or weighted activity measures). In some implementations, a matrix $\Sigma$ is defined as $\Sigma = diag(var(\beta))$. Next, the network scoring engine 114 uses the structure of the relevant network to generate a Laplacian matrix (e.g., as described above). The network may be weighted, signed, and directed, or any combination thereof. The network scoring engine 114 may solves the Laplacian expression of Eq. 12 with the left hand side equal to zero to generate $f_2$ (the vector of activity values). The network scoring engine 114 then may compute the variance of the vector of activity values. In some implementations, this vector is calculated in accordance with

$$\mathrm{var}\left(f_2\right) = L_3^{-1}L_2^T\Sigma L_2\left(L_3^{-1}\right)^T \tag{15}$$

where $L_2$ and $L_3$ are as defined in Eq. 11. The network scoring engine 114 may then compute the confidence intervals of each entry of $f_2$ in accordance with

$$f_2\left(x\right) \pm z\left(1-\alpha/2\right)\sqrt{\mathrm{var}\left(f_2\left(x\right)\right)} \tag{16}$$

where $z(1-\alpha/2)$ is the associated N(0,1) quantile (e.g., 1.96 if $\alpha=0.05$). The network scoring engine 114 may then compute the quadratic form matrix used to compute an NPA score. In some implementations, the quadratic form matrix is computed in accordance with Eq. 3, above. The network scoring engine 114 then may compute an NPA score using the quadratic form matrix $Q$ in accordance with:

$$NPA = f_2^T Q f_2 \,. \tag{17}$$

The network scoring engine 114 then may compute a variance of the NPA score. In some implementations, this variance is computed in accordance with

$$\mathrm{var}\left(NPA\right) = \mathrm{var}\left(f_2^T Q f_2\right) = 2tr\left(Q\Psi^2 Q\Psi^2\right) + 4f_2^T Q\Psi^2 Q f_2 \tag{18}$$

where $\Psi = \mathrm{var}(f_2)$. The network scoring engine 114 then may compute a confidence interval for the NPA score. In some implementations, the confidence interval is computed in accordance with

$$NPA \pm z\left(1-\frac{\alpha}{2}\right)\sqrt{\mathrm{var}\left(NPA\right)} \tag{19}$$

or

$$NPA \pm \sqrt{1/\left(1-\alpha\right)}\sqrt{\mathrm{var}\left(NPA\right)}\,. \tag{20}$$

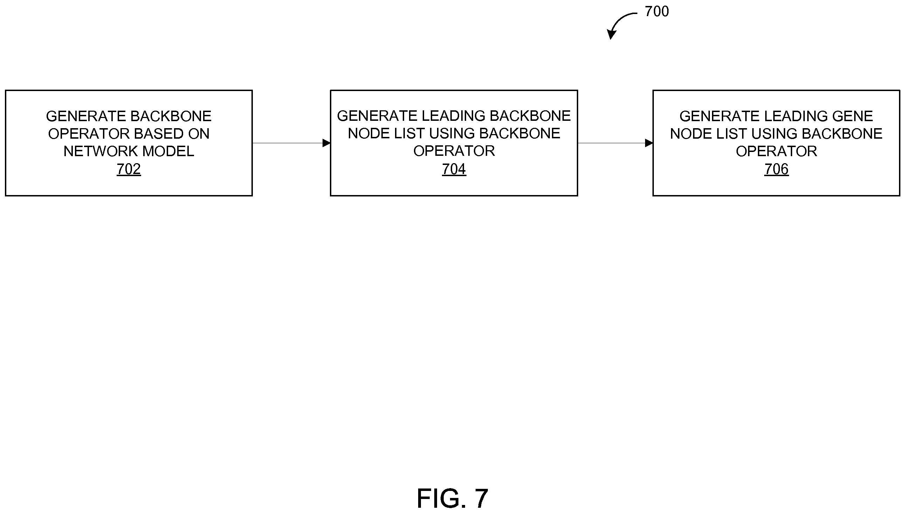

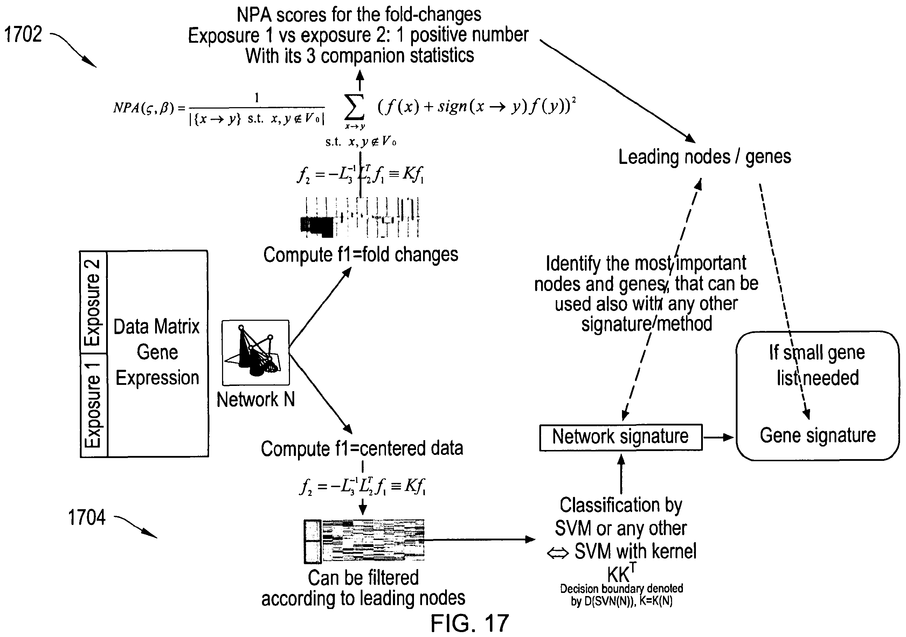

FIG. 7 is a flow diagram of an illustrative process for identifying leading backbone and gene nodes, which is illustrated by the computational path 1702 of FIG. 17. At step 702, the network scoring engine 114 generates a backbone operator based on the identified network model. The backbone operator acts on a vector of the activity measures of the supporting nodes and outputs a vector of activity values for the backbone nodes. A suitable backbone operator in some implementations is the operator K defined above in Eq. 13.

At step 704, the network scoring engine 114 generates a list of leading backbone nodes using the backbone operator generated at step 702. The leading backbone nodes may represent the most significant backbone nodes identified during the analysis of the treatment and control data and the causal biological network model. To generate this list, the network scoring engine 114 may use the backbone operator to form a kernel that can then be used in an inner product between the vector of activity values for the backbone nodes and itself. In some implementations, the network scoring engine 114 generates the list of leading backbone nodes by ordering the terms in the sum that results from such an inner product in decreasing order, and selecting either a fixed number of the nodes corresponding to the largest contributors to the sum or the number of the most significantly contributing nodes required to achieve a specified percentage of the total sum *(e.g.,* 60%). Equivalently, the network scoring engine 114 may generate the leading backbone nodes list by including the backbone nodes that make up 80% of the NPA score by computing the cumulative sum of the ordered terms of Eq. 1. As discussed above, this cumulative sum can be calculated as the cumulative sum of the terms of the following inner product (using the backbone operator *K*):

$$f_1^T K^T K f_1$$  (21)

Thus, the identification of leading nodes depends both on activity measures and network topology.

At step 706, the network scoring engine 114 generates a list of leading gene nodes using the backbone operator generated at step 702. As shown by Eq. 2, an NPA score may be represented as a quadratic form in the fold-changes. Thus, in some implementations, a leading gene list is generated by identifying the terms of the ordered sum of the following scalar product:

$$\left\langle f_1 \middle| L_2 (L_3^{-1})^T L_3^{-1} L_2^T f_1 \right\rangle$$  (22)

Both ends of a leading gene list may be important as the genes contributing negatively to the NPA score also have biological significance.

In some implementations, the network scoring engine 114 also generates a structural importance value for each gene at step 706. The structural importance value is independent of the experimental data and represents the fact that some genes might be more important to inferring the value of the backbone nodes than others due to the gene's position in the model. The structural importance may be defined for gene j by

$$I_j = \sum_{i=1}^{N} \left| (L_3^{-1} L_2^T)_{ij} \right|$$  (23)

The biological entities in the leading backbone node list and the genes in the leading gene node list are candidates for biomarkers of activation of the underlying networks by the treatment condition (relative to the control condition). These two lists may be used separately or together to identify targets for future research, or may be used in other biomarker identification processes, as described below.

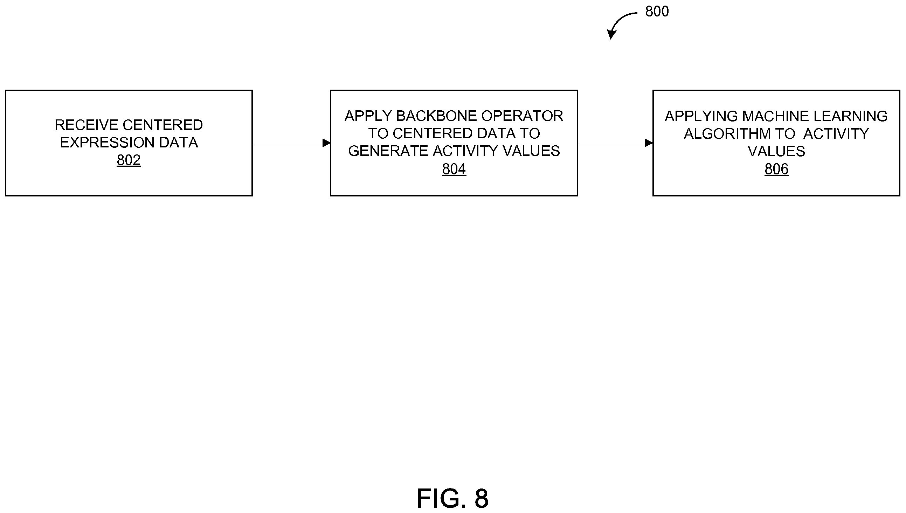

FIG. 8 is a flow diagram of an illustrative process for classifying backbone node activity values, which is illustrated by the computational path 1704 of FIG. 17. At step 802, the network scoring engine 114 receives centered expression data for the supporting entities in a biological system. This centered expression data is data taken from individual samples that has been centered by subtracting the population mean for such data. Thus, the centered data received at step 802 will include both positive and negative values representing deviations above and below the population mean, respectively.

At step 804, the network scoring engine 114 applies a backbone operator (as described above with respect to the calculation of the NPA score) to generate activity values for the backbone nodes based on the centered expression data. A suitable backbone operator in some implementations is the operator K defined above in Eq. 13. The result of step 804 is to take centered expression data representative of the supporting entities and generate activity values representative of the unobserved backbone entities. In many applications, the number of supporting entities is far larger than the number of backbone entities in a given network model, and thus by executing step 804, the network scoring engine reduces the dimensionality of the problem from a space that is the size of the number of supporting entities to a space that is the size of the number of backbone entities.

At step 806, the network scoring engine 114 applies a machine learning algorithm to the activity values generated at step 804 to generate a classifier that distinguishes activity values from samples of a particular biological class *(e.g., a particular phenotype)* from samples of another biological class. The network scoring engine 114 may use any one or more known machine-learning algorithms at step 806, including but not limited to support vector machine techniques, linear discriminant analysis techniques, Random Forest techniques, k-nearest neighbors techniques, partial least squares techniques (including techniques that combine partial least squares and linear discriminant analysis features), logistic regression techniques, neural network-based techniques, decision tree-based techniques and shrunken centroid techniques (e.g., as described by Tibshirani, Hastle, Narasimhan and Chu in "Diagnosis of multiple cancer types by shrunken centroids of gene expression," Proc. Natl. Acad. Sci. , v. 99, n. 10, 2002). A number of such techniques are available as packages for the R programming language, including lda, svm, randomForest, knn, pls.lda and pamr.

In some implementations, the network scoring engine 114 uses K as the backbone operator at step 804 and SVM as the machine learning algorithm applied at step 806. An alternative implementations that will achieve the same classifier at the conclusion of step 806 is one in which the network scoring engine 114 is configured to apply an SVM to the centered expression data (of step 802) directly, but using the backbone operator $K$ to form the kernel $KK^T$ of the SVM.

Not all of the backbone nodes and corresponding activity values may be used at step 806 to generate a classifier. In some implementations, only the leading nodes identified using the technique described above with reference to FIG. 7 are used, with the remaining backbone nodes ignored.

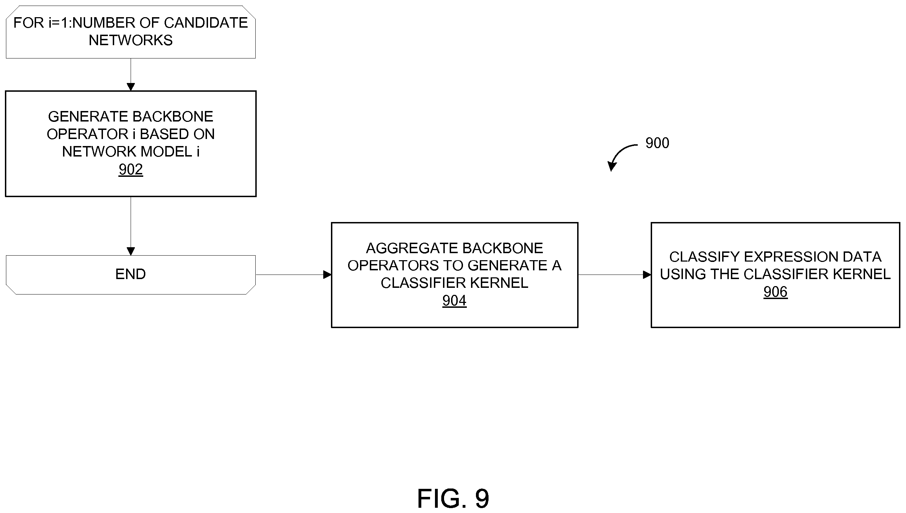

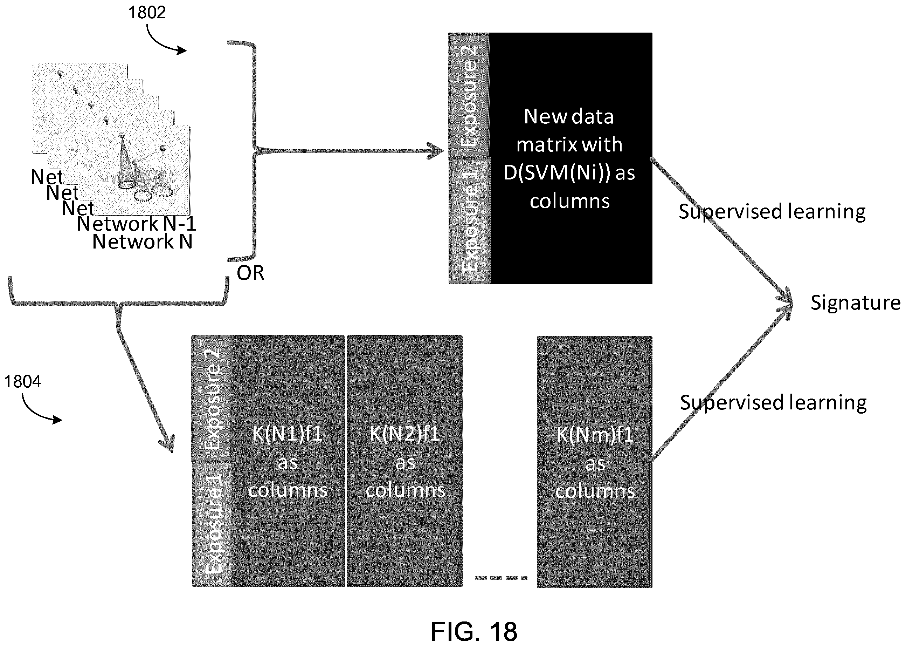

FIG. 9 is a flow diagram of an illustrative process for identifying a feature space from multiple networks for use in identifying entities for biomarkers, which is illustrated by the computational path 1804 of FIG. 18. The network scoring engine 114 iterates step 902 for each network model in a set of network models (e.g., the set of those that have been identified as potentially relevant to a biological phenomenon of interest). At step 902, the network scoring engine 114 generates a backbone operator based on a network model. As described above with reference to FIG. 7, one suitable backbone operator is the operator K of Eq. 13. At step 904, the network scoring engine 114 aggregates the backbone operators generated at the iterations of step 902 into a kernel for use in a classification technique, such as SVM. In some implementations, the kernel generated at step 904 is based on several backbone operators, each corresponding to a different network model. These several backbone operators may be combined via a weighted average or by a non-linear function. For example, several backbone operators may be combined via a kernel alignment technique. In some implementations, the network scoring engine 114 aggregates the backbone operators at step 904 using the P-values of the two perturbation tests described above. For example, the network scoring engine 114 may take a linear combination of the kernels of the backbone operators with weights that are equal to 1 when both perturbation tests give results below 0.05 and 0 otherwise. In other examples, other functions of the perturbation test statistics or other statistics may be used to generate weights for a linear combination (e.g., a sigmoid function or an average -log10 function), reflecting various preferences for the emphasis to be placed on various ones of the statistics in the weighted combination. In some implementations, the kernel generated at step 904 is the solution to a semidefinite programming problem that seeks to optimize the value of the kernel to minimize an objective function. Many such approaches are known in the literature. In some implementations, the network scoring engine 114 generates the kernel at step 904 by stacking several kernels (based on backbone operators) to form a new feature space that includes all of the backbone components of each of the corresponding networks.

At step 906, the network scoring engine 114 generates a classifier using the kernel of step 904 and the activity values of the backbone nodes (which may be calculated in any of the ways described herein). Any of a number of known techniques may be used to generate a classifier based on a kernel that defines an inner product in a feature space, such as a support vector machine technique.

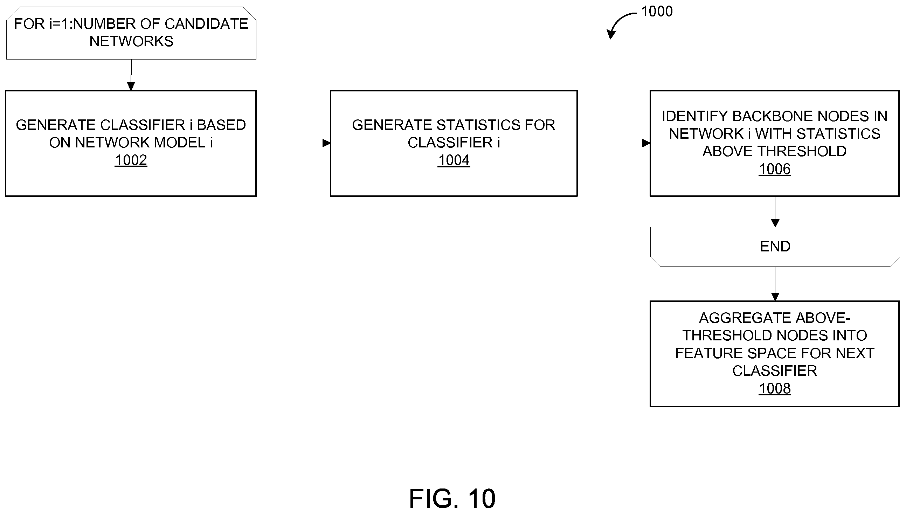

FIG. 10 is a flow diagram of an illustrative process for identifying a feature space from multiple classifiers for use in identifying entities for biomarkers, which is illustrated by the computational path 1802 of FIG. 18. For each of a number of candidate networks (which may represent, for example, a number of different biological mechanisms hypothesized to play a role in a phenomenon of interest), the network scoring engine 114 performs the following steps. At step 1002, the network scoring engine 114 generates a classifier for the network model based on the experimental data. The network scoring engine 114 may use any of the machine learning techniques described herein to generate the classifier at step 902, including SVM. At step 1004, the network scoring engine 114 generates statistics descriptive of the performance of the classifier generated at step 1002. Statistics descriptive of a classifier's performance includes the cross-validation accuracy of the classifier and the decision values corresponding to each backbone node. At step 1006, the network scoring engine 114 identifies backbone nodes in the network model whose associated statistics indicate that the significance of the backbone nodes exceeds a threshold. In some implementations, step 1006 is omitted, and all backbone nodes are used. At step 1008, the network scoring engine 114 aggregates the above-threshold backbone nodes across network models into a feature space that can be used as the basis for a new classifier using any known classification technique (e.g., a machine-learning technique such as SVM). One advantage of performing a classification on the space of backbone node activity values is that the dimension of this space is typically much smaller than the dimension of the supporting entity space (e.g., tens of backbone nodes as compared to several thousand measured genes).

In applications in which a list of significant genes or other supporting entities are desired (rather than a list of significant backbone entities), the network scoring engine 114 may be configured to further process the results of the classification techniques described herein which generate classifiers in backbone space in order to generate classifiers in gene space. For example, if the network scoring engine 114 generates a classifier in backbone node space according to any of the techniques described herein, the network scoring engine 114 may also be configured to calculate a measure of the relative importance of different genes to the classifier by taking the scalar product of the value of the decision function for the classifier evaluated at a particular activity measure for the gene of interest and the gradient of the decision function evaluated at that activity measure. The network scoring engine 114 may compare the result of this calculation across genes (or other supporting entities) to determine which play the most important role in the outcome of the decision function.

In some applications, a backbone node list that can be used for classification purposes may be generated a single node at a time. For example, the network scoring engine 114 may be configured to identify a single backbone node (e.g., the backbone node with the highest activity value) and use only the value of that node as the basis for a computational classifier (using any machine learning technique). The network scoring engine 114 may then select a second node (e.g., a backbone node with the second highest activity value) and use the value of both nodes as the basis for a computational classifier. This process may continue, with the network scoring engine 114 evaluating the covalidation accuracy at each iteration, until a desired number of backbone nodes is reached or a desired accuracy is reached.

FIG. 11 is a flow diagram of an illustrative process for identifying backbone nodes for use in a classification system based on F-statistics. The network scoring engine 114 iterates steps 1102-1116 for each network model in a set of network models (e.g., the set of those that have been identified as potentially relevant to a biological phenomenon of interest). The discussion of FIG. 11 refers to the network corresponding to the current iteration as the "current network." At step 1102, the network scoring engine 114 receives a set of centered expression data *(e.g., as described above with reference to FIG. 8)*. At step 1104, the network scoring engine 114 applies a backbone operator associated with the current network (such as the backbone operator K) to the centered expression data to generate activity values (e.g., as described above with reference to FIG. 8). At step 1106, the network scoring engine 114 sorts the z-scores of the activity values according to the order of the F-statistic. At step 1108, the network scoring engine 114 generates a value $p_{gs}$ that represents the mean-rank enrichment P-values of the backbone nodes in the current network. At step 1110, the network scoring engine 114 generates intermediate cumulative sums of the ordered Z-scores, and at step 1012, recomputes the F-test statistic for each intermediate cumulative sum. At step 1114, the network scoring engine 114 selects the first intermediate cumulative sum whose F-test value is larger than the F-test value of the following intermediate cumulative sum *(i.e., just before the F-test values begin to decrease)*. At step 1116, the network scoring engine 114 outputs the set of backbone nodes in the current network whose Z-scores are included in the cumulative sum. Once steps 1102-1116 have been executed for each network model in the set of network models, the network scoring engine 114 creates a matrix that aggregates the activity values of all of the backbone nodes selected at the various iterations of step 1116 for network models whose associated value $p_{gs}$ does not exceed a predetermined threshold $p_0$. A machine learning algorithm, such as any of those described herein, may then be applied to the matrix.

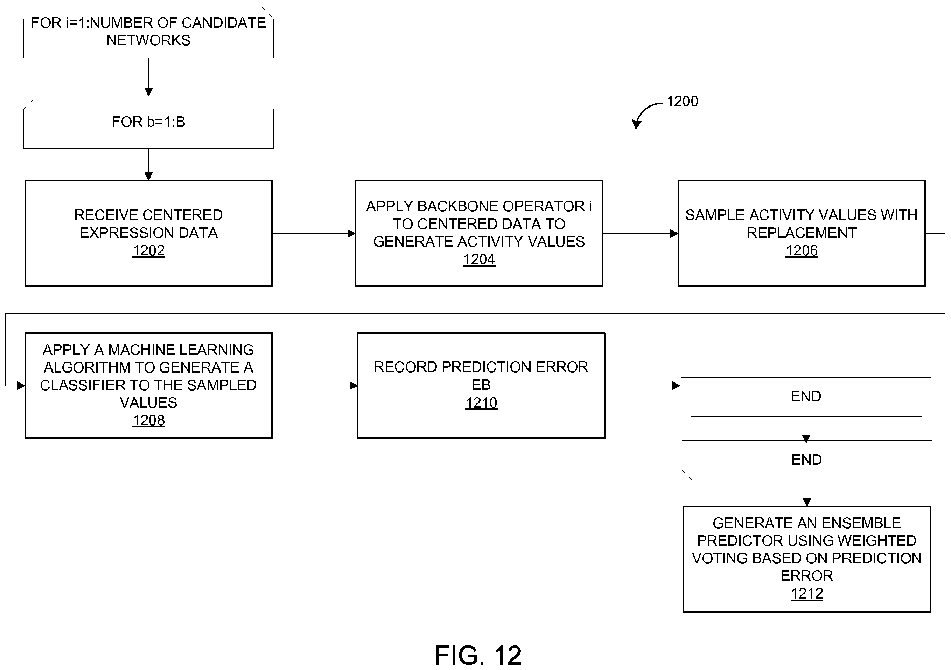

FIG. 12 is a flow diagram of an illustrative process for generating an ensemble predictor from backbone node activity values. The network scoring engine 114 iterates steps 1202-1210 for each network model in a set of network models (e.g., the set of those that have been identified as potentially relevant to a biological phenomenon of interest). The discussion of FIG. 12 refers to the network corresponding to the current iteration as the "current network." In addition, the network scoring engine iterates steps 1202-1210 a given number B of times for each network model. At step 1202, the network scoring engine 114 receives a set of centered expression data *(e.g., as described above with reference to FIG. 8)*. At step 1204, the network scoring engine 114 applies a backbone operator associated with the current network (such as the backbone operator K) to the centered expression data to generate activity values (e.g., as described above with reference to FIG. 8). At step 1206, the network scoring engine 114 samples the activity values generated at step 1204 with replacement. In some implementations, 80% of the total number of gene activity values are sampled with replacement *(i.e., as part of a bootstrapping technique)*. A percentage of the data sets (each of which may correspond, for example, to a particular patient) are also sampled *(e.g., 20%)*. At step 1208, the network scoring engine 114 applies a machine learning algorithm to generate a classifier based on the sample values. The machine learning algorithm may include any of those described herein. At step 1210, the network scoring engine 114 records the prediction error associated with the classifier generated at step 1208 *(e.g., by evaluating the classifier on a test data set whose classification is known)*. Once the network scoring engine has executed steps 1202-1210 B times for each network, the network scoring engine 114 generates an ensemble predictor which uses a weighted voting scheme to classify activity values. In some implementations, the weights depend on the prediction errors calculated at step 1210. For example, if the prediction error for a particular iteration is represented by $e_b$, the network scoring engine 114 may calculate the weight for that iteration in accordance with:

$$w_b = \log\left(\frac{1 - e_b}{e_b}\right),$$

(24)

where $0 \le e_b \le 1$. In some implementations, the network scoring engine 114 calculates the weight for an iteration in accordance with:

$$w_b = \begin{cases} \log\left(\dfrac{1-e_b}{e_b}\right) & 0 \le e_b < 0.5 \\ 0 & e_b \ge 0.5 \end{cases} \qquad (25)$$

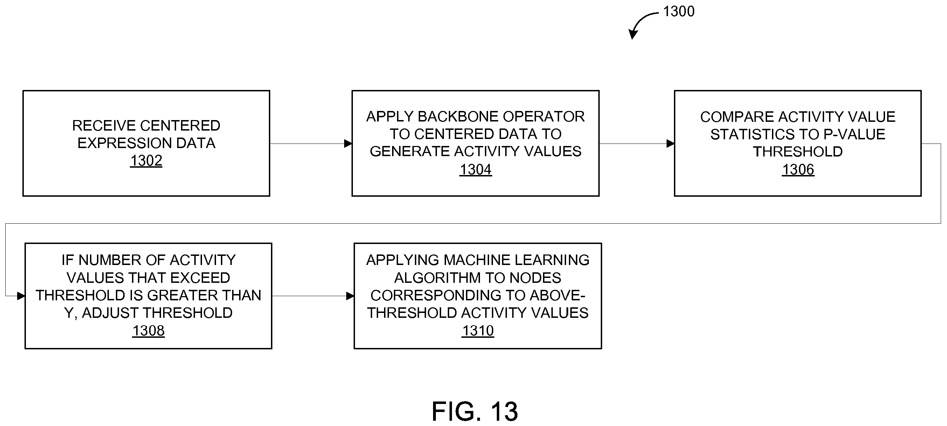

FIG. 13 is a flow diagram of an illustrative process for identifying backbone nodes for use in a classification system based on p-values. At step 1302, the network scoring engine 114 receives a set of centered expression data (e.g., as described above with reference to FIG. 8). At step 1304, the network scoring engine 114 applies a backbone operator associated with the current network (such as the backbone operator K) to the centered expression data to generate activity values (e.g., as described above with reference to FIG. 8). At step 1306, the network scoring engine 114 compares the p-values associated with the activity values generated at step 1304 with a predetermined threshold p-value. At step 1308, the network scoring engine 114 determines whether the number of activity values with p-values below the threshold exceeds a predetermined number Y; if so, the network scoring engine increases the threshold and repeats step 1306. In some implementations, the network scoring engine 114 determines whether the number of activity values with p-values below the threshold falls below the predetermined number Y; if so, the network scoring engine decreases the threshold and repeats step 1306. At step 1310, the network scoring engine 114 applies a machine learning algorithm to the activity values of backbone nodes corresponding to p-values that exceed the threshold. Any of the machine learning algorithms described herein may be used.

Implementations of the present subject matter can include, but are not limited to, systems methods and computer program products comprising one or more features as described herein as well as articles that comprise a machine-readable medium operable to cause one or more machines (e.g., computers, robots) to result in operations described herein. The methods described herein can be implemented by one or more processors or engines residing in a single computing system or multiple computing systems. Such multiple computing systems can be connected and can exchange data and/or commands or other instructions or the like via one or more connections, including but not limited to a connection over a network (e.g. the Internet, a wireless wide area network, a local area network, a wide area network, a wired network, or the like), via a direct connection between one or more of the multiple computing systems.

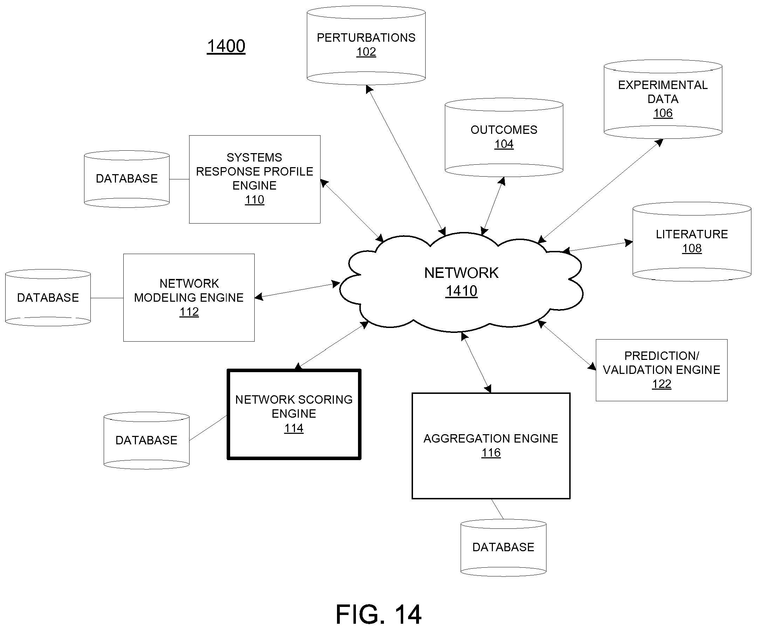

FIG. 14 is a block diagram of a distributed computerized system 1400 for quantifying the impact of biological perturbations. The components of the system 1400 are the same as those in the system 100 of FIG. 1, but the arrangement of the system 100 is such that each component communicates through a network interface 1410. Such an implementation may be appropriate for distributed computing over multiple communication systems including wireless communication system that may share access to a common network resource, such as "cloud computing" paradigms.

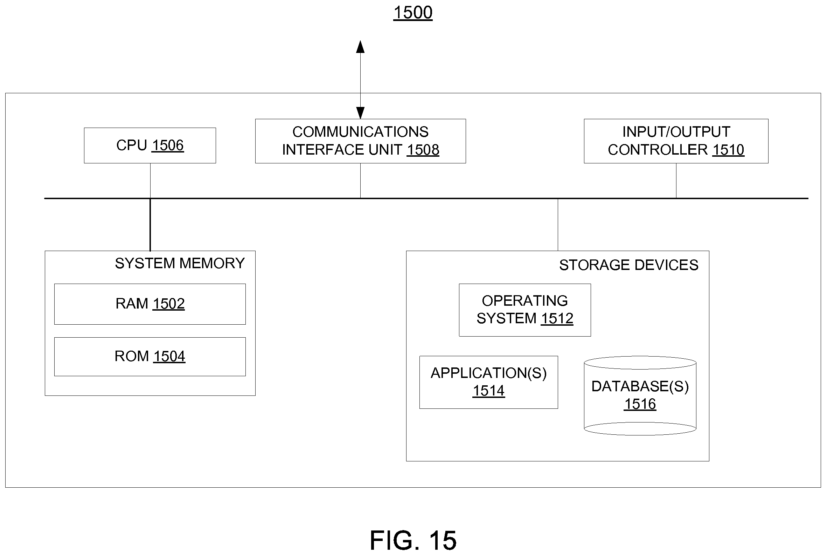

FIG. 15 is a block diagram of a computing device, such as any of the components of system 100 of FIG. 1, for performing processes described with reference to any of the figures herein. Each of the components of system 100, including the SRP engine 150, the network modeling engine 152, the network scoring engine 154, the aggregation engine 156 and one or more of the databases including the outcomes database, the perturbations database, and the literature database may be implemented on one or more computing devices 1500. In certain aspects, a plurality of the above-components and databases may be included within one computing device 1500. In certain implementations, a component and a database may be implemented across several computing devices 1500.

The computing device 1500 comprises at least one communications interface unit, an input/output controller 1510, system memory, and one or more data storage devices. The system memory includes at least one random access memory (RAM 1502) and at least one read-only memory (ROM 1504). All of these elements are in communication with a central processing unit (CPU 1506) to facilitate the operation of the computing device 1500. The computing device 1500 may be configured in many different ways. For example, the computing device 1500 may be a conventional standalone computer or alternatively, the functions of computing device 1500 may be distributed across multiple computer systems and architectures. The computing device 1500 may be configured to perform some or all of modeling, scoring and aggregating operations. In FIG. 15, the computing device 1500 is linked, via network or local network, to other servers or systems.

The computing device 1500 may be configured in a distributed architecture, wherein databases and processors are housed in separate units or locations. Some such units perform primary processing functions and contain at a minimum a general controller or a processor and a system memory. In such an aspect, each of these units is attached via the communications interface unit 1508 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SAS™, ATP, BLUETOOTH™, GSM and TCP/IP.

The CPU 1506 comprises a processor, such as one or more conventional microprocessors and one or more supplementary co-processors such as math co-processors for offloading workload from the CPU 1506. The CPU 1506 is in communication with the communications interface unit 1508 and the input/output controller 1510, through which the CPU 1506 communicates with other devices such as other servers, user terminals, or devices. The communications interface unit 1508 and the input/output controller 1510 may include multiple communication channels for simultaneous communication with, for example, other processors, servers or client terminals. Devices in communication with each other need not be continually transmitting to each other. On the contrary, such devices need only transmit to each other as necessary, may actually refrain from exchanging data most of the time, and may require several steps to be performed to establish a communication link between the devices.

The CPU 1506 is also in communication with the data storage device. The data storage device may comprise an appropriate combination of magnetic, optical or semiconductor memory, and may include, for example, RAM 1502, ROM 1504, flash drive, an optical disc such as a compact disc or a hard disk or drive. The CPU 1506 and the data storage device each may be, for example, located entirely within a single computer or other computing device; or connected to each other by a communication medium, such as a USB port, serial port cable, a coaxial cable, an Ethernet type cable, a telephone line, a radio frequency transceiver or other similar wireless or wired medium or combination of the foregoing. For example, the CPU 1506 may be connected to the data storage device via the communications interface unit 1508. The CPU 1506 may be configured to perform one or more particular processing functions.

The data storage device may store, for example, (i) an operating system 1512 for the computing device 1500; (ii) one or more applications 1514 (e.g., computer program code or a computer program product) adapted to direct the CPU 1506 in accordance with the systems and methods described here, and particularly in accordance with the processes described in detail with regard to the CPU 1506; or (iii) database(s) 1516 adapted to store information that may be utilized to store information required by the program. In some aspects, the database(s) includes a database storing experimental data, and published literature models.

The operating system 1512 and applications 1514 may be stored, for example, in a compressed, an uncompiled and an encrypted format, and may include computer program code. The instructions of the program may be read into a main memory of the processor from a computer-readable medium other than the data storage device, such as from the ROM 1504 or from the RAM 1502. While execution of sequences of instructions in the program causes the CPU 1506 to perform the process steps described herein, hard-wired circuitry may be used in place of, or in combination with, software instructions for implementation of the processes of the present disclosure. Thus, the systems and methods described are not limited to any specific combination of hardware and software.

Suitable computer program code may be provided for performing one or more functions in relation to modeling, scoring and aggregating as described herein. The program also may include program elements such as an operating system 1512, a database management system and "device drivers" that allow the processor to interface with computer peripheral devices (e.g., a video display, a keyboard, a computer mouse, *etc.)* via the input/output controller 1510.

A computer program product comprising computer-readable instructions is also provided. The computer-readable instructions, when loaded and executed on a computer system, cause the computer system to operate according to the methods, or one or more steps of the methods described above. The term "computer-readable medium" as used herein refers to any non-transitory medium that provides or participates in providing instructions to the processor of the computing device 1500 (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 1506 (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 1500 *(e.g.,* a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

The systems and methods described herein have been applied to the problem of identifying biomarkers for predicting the response of patients with ulcerative colitis to anti-TNFa treatment, and in particular, infliximab (an anti-inflammatory antibody). Clinical trials showed that induction with 5 mg/kg gives a clinical response in 64% to 69% of patients. However, clinicians have been advised to balance the potentially beneficial use of infliximab against the possibility of complications of autoimmunity, opportunistic infection, sepsis, and malignancy. To generate a signature that may distinguish between patients who should and should not receive this therapy, data from the literature from two cohorts of patients who received a treatment with infliximab for refractory ulcerative colitis was used. In this data set, gene profiling from colonic biopsies was performed with Affymetrix HGU-133 Plus 2.0 Arrays (GSE 12251 and GSE 14580).

To evaluate the performance of certain implementations of the systems and methods described herein, each patient data set was compared to data averaged across all non-responding patients, and these comparisons were used to determine a network perturbation of the TNF-IL1-NF$_K$B model, which was then used as the input for finding a mechanistic signature differentiating responders from non-responders. A nearest shrunken centroid technique was also used during classification, as described by Tibshirani et al. in "Diagnosis of multiple cancer types by shrunken centroids of gene expression," Proc. Natl. Acad. Sci. 2002, 99:6567-6572.

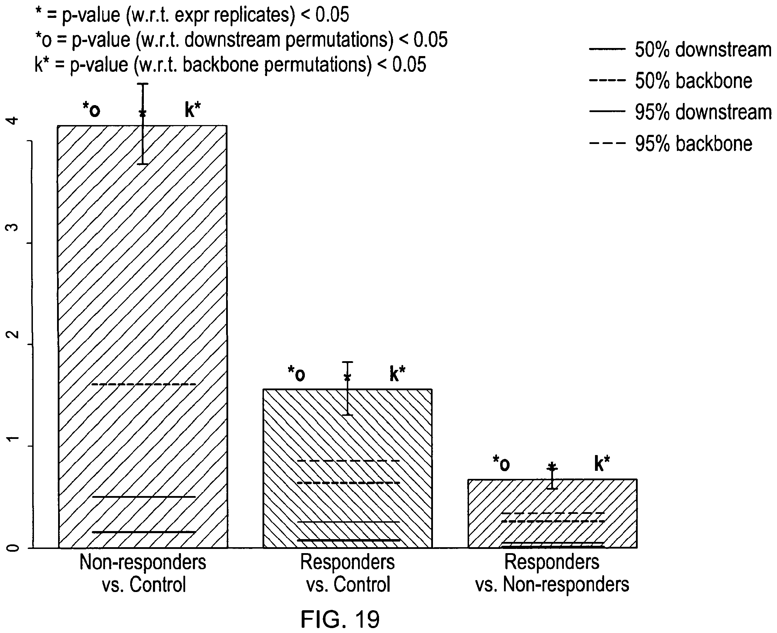

FIG. 19 is a graph depicting NPA scores for various treatment/control conditions. In particular, FIG. 19 shows NPA scores calculated for the TNF-IL1-NF$_K$B network model when the input represented fold-changes for the following treatment/control combinations: non-responder/control, responder/control, and responder/non-responder. It can be seen that the NPA score for the non-responder/control comparison is much higher than the scores for either the responder/control and responder/non-responder comparisons, indicating that the TNF-IL1-NF$_K$B network model represents a biological mechanism that may usefully differentiate responders from non-responders.

To determine what mechanisms may be especially relevant in distinguishing responders from non-responders, the activity values for the backbone nodes is analyzed. For each of the backbone nodes RNF, IL1R1, MYD88, catof(IL1R1) and catof(MYD88), the activity value generated for each of the three treatment/control conditions is compared (i.e., non-responder/control, responder/control, and responder/non-responder). The backbone nodes correspond to the second subset of nodes (as described in the computer-implemented methods), representing biological entities, i.e., backbone entities, whose activities are not physically measured. By comparing the magnitude of the activity values for each of these backbone entities, the system 100 is able to generate several potential biomarkers and corresponding hypotheses. First, the system 100 identified TNF as useful for distinguishing ulcerative colitis ("UC") patients from controls, but not for distinguishing responders from non-responders. ILR1 is useful for distinguishing non-responders from controls and from responders, but not for distinguishing responders from controls. The system 100 further identified MYD88 is useful for distinguishing responders from non-responders as well as distinguishing UC patients from controls.

The system 100 did not identify TNF nor IL1R1 as distinguishing the treatment outcomes, but did identify MYD88 as distinguishing the outcomes.

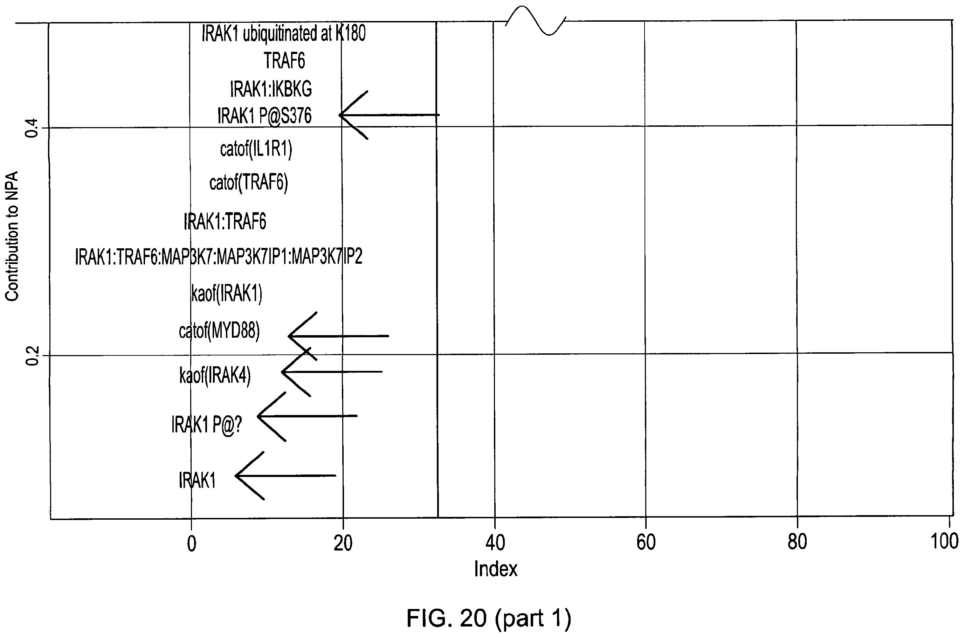

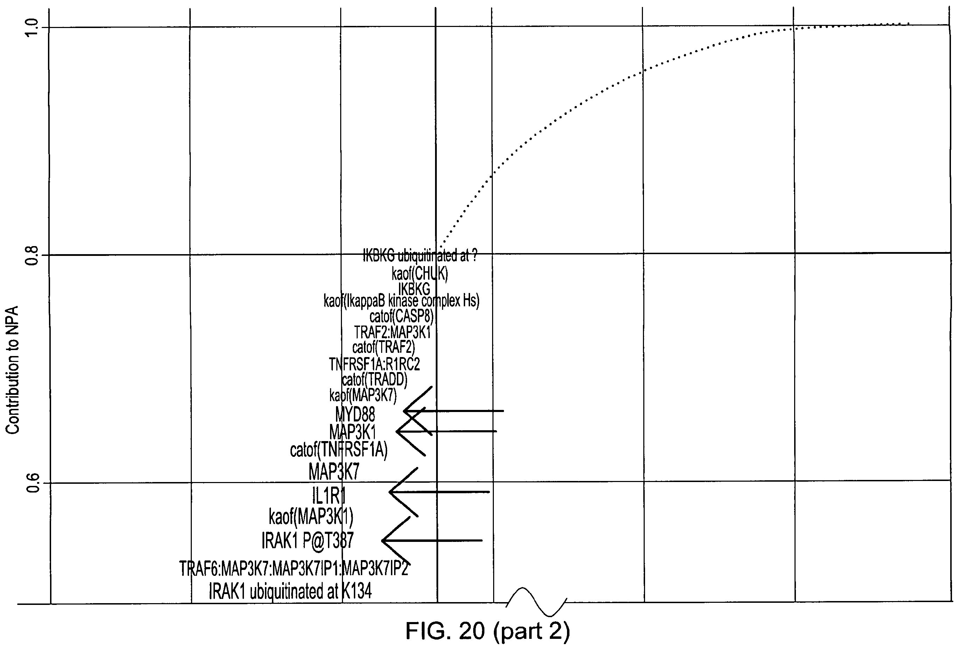

FIG. 20 illustrates a leading backbone node list for the TNF-IL1-NF$_K$B network model generated by the system 100 when supplied with the responder/non-responder fold-change data set. The backbone entities are listed from bottom to top in order of the magnitude of their contribution to the NPA score sum, as described above. Of the top entities, those with arrows were also identified as significant to the network using a PAM technique, indicating good agreement between previous work and the results of the systems and methods described herein. Accordingly, the systems and methods described herein provide a network model relating to the simulation of the biology of actions of TNF, IL1 and NF$_K$B wherein the backbone nodes comprise MYD88, MAP3K1, IL1R, IRAK1 P@T387, IRAK P@S376, catof(MYD88), kaof(IRAK4), IRAK1 P@? and IRAK1.

## Claims

1. A computerized method for generating a classifier for phenotypic prediction, comprising the steps of:

   (a) receiving, at a processing device, data indicative of a computational causal network model, wherein the data is representative of a biological system that contributes to a phenotype and includes:

      a plurality of nodes that represent biological entities in the biological system; and
      a plurality of edges connecting pairs of nodes among the plurality of nodes and representing relationships between the biological entities represented by the nodes;
      wherein one or more edges is associated with a direction value that represents a causal activation or causal suppression relationship between the biological entities represented by the nodes, and
      wherein each node is connected by an edge to at least one other node;

(b) receiving, at the processing device, (i) a first set of data corresponding to activities of a first subset of biological entities obtained under a first set of conditions; and (ii) a second set of data corresponding to activities of the first subset of biological entities obtained under a second set of conditions different from the first set of conditions, wherein the first and second sets of conditions relate to the phenotype;

(c) calculating, with the processing device, a set of activity measures for a first subset of nodes corresponding to the first subset of biological entities, each activity measure representing a difference between a first measurement under the first set of conditions from b(i) and a second measurement under the second set of conditions from b(ii);

(d) generating, with the processing device, an inferred set of activity values for a second subset of nodes representing candidates of biological entities that contribute to the phenotype but whose activities are not measured, by identifying, for each particular node in the second subset of nodes, an activity value that optimizes a difference statement that represents the difference between the activity value of the particular node and the activity value or activity measure of nodes to which the particular node is connected by an edge within the computational causal network model, wherein the difference statement depends on the activity values of each node in the second subset of nodes, and wherein each activity value in the set of activity values is a linear combination of activity measures in the set of activity measures; and

(e) generating, with the processing device using a machine learning technique, a classifier for the phenotype using both the set of activity measures and the set of activity values as inputs.

2. The computerized method of claim 1, wherein steps (a), (b), (c) and (d) are performed for a plurality of computational causal network models, and the sets of activity values corresponding to each of the computational causal network models are aggregated into a matrix containing the set of activity values to be used at step (e) by the machine learning technique.

3. The computerized method of claim 2, wherein steps (a), (b), (c), (d) and (e) are performed for a plurality of computational causal network models, and further comprising:

(h1) for each classifier, identifying one or more biological entities of the second set of biological entities with classification performance statistics above a threshold across the plurality of computational causal network models; and

(h2) aggregating all of the identified biological entities into a set of high performing entities;

(h3) generating, with the processing device, a new classifier of biological condidtions based on the activity values associated with the set of high performing entities using a machine learning technique; and

(h4) outputting the new classifier.

4. The computerized method of any of claims 1-3, wherein the machine learning technique includes a support vector machine technique.

5. The computerized method of any of claims 1-4, wherein the difference statement further depends on the direction values of the edges between each node in the second subset of nodes and the nodes to which each node in the second subset of nodes is connected.

6. The computerized method of any of claims 1-5, wherein the linear combination depends on edges between nodes in the first subset of nodes and nodes in the second subset of nodes, and also depends on edges between nodes in the second subset of nodes.

7. The computerized method of any of claims 1-5, wherein the linear combination does not depend on edges between nodes in the first subset of nodes.

8. The computerized method of any of claims 1-7, wherein the activity measure of step (c) is a fold-change value, and the fold-change value for each node in the first subset of nodes represents a logarithm of the difference between corresponding sets of treatment data for the biological entity represented by the respective node.

9. The computerized method of any of claims 1-8, wherein the first subset of biological entities includes a set of genes and the first set of data include expression levels of the set of genes.

10. A computer program product comprising computer-readable instructions that, when executed in a computerized system comprising at least one processor, cause the processor to carry out one or more steps of the method of any of claims 1-9.

11. A computerized system comprising a processing device configured with non-transitory computer-readable instructions that, when executed, cause the processing device to carry out the method of any of claims 1-9.

12. The computerized method of any of claims 1-9, wherein the optimization of the difference statement comprises maximizing the difference statement, minimizing the difference statement, or making the difference statement as close as possible to a target value.

**Patentansprüche**

1. Computerisiertes Verfahren zur Erzeugung eines Klassifikators zur phänotypischen Vorhersage, aufweisend die Schritte:

(a) Empfangen, an einer Verarbeitungsvorrichtung, von ein rechnergestütztes kausales Netzwerkmodell angebenden Daten, wobei die Daten repräsentativ für ein zu einem Phänotyp beitragendes, biologisches System sind, und beinhalten:

eine Vielzahl von biologische Entitäten in dem biologischen System darstellenden Knoten; und
eine Vielzahl von Paaren von Knoten aus der Vielzahl von Knoten verbindenden und Beziehungen zwischen den biologischen, durch die Knoten dargestellten Entitäten darstellenden Kanten;
wobei eine oder mehrere Kanten einem eine kausale Aktivierungsoder kausale Unterdrückungsbeziehung zwischen den biologischen, durch die Knoten dargestellten Entitäten darstellenden Richtungswert zugewiesen sind, und wobei jeder Knoten durch eine Kante mit wenigstens einem anderen Knoten verbunden ist;

(b) Empfangen, an der Verarbeitungsvorrichtung, (i) eines ersten, Aktivitäten einer ersten Teilmenge von biologischen Entitäten, die unter einem ersten Satz von Bedingungen erhalten werden, entsprechenden Datensatzes; und (ii) eines zweiten, Aktivitäten der ersten Teilmenge von biologischen Entitäten, die unter einem zweiten Satz von sich von dem ersten Satz von Bedingungen unterscheidenden Bedingungen erhalten werden, entsprechenden Datensatzes, wobei sich der erste und der zweite Satz von Bedingungen auf den Phänotyp beziehen;
(c) Berechnen, durch die Verarbeitungsvorrichtung, eines Satzes von Aktivitätsmaßen für eine erste, der ersten Teilmenge von biologischen Entitäten entsprechenden Teilmenge von Knoten, wobei jedes Aktivitätsmaß eine Differenz zwischen einer ersten Messung aus dem ersten Satz von Bedingungen von b(i) und einer zweiten Messung aus dem zweiten Satz von Bedingungen von b(ii) darstellt;
(d) Erzeugen, mit der Verarbeitungsvorrichtung, eines abgeleiteten Satzes von Aktivitätswerten für eine zweite Teilmenge von Kandidaten biologischer, zum Phänotyp beitragenden Entitäten, deren Aktivitäten jedoch nicht gemessen werden, darstellenden Knoten, durch Identifizieren, für jeden bestimmten Knoten in der zweiten Teilmenge von Knoten, eines eine Differenzaussage optimierenden Aktivitätswerts, die die Differenz zwischen dem Aktivitätswert des bestimmten Knotens und dem Aktivitätswert oder dem Aktivitätsmaß von Knoten darstellt, mit denen der bestimmte Knoten durch eine Kante innerhalb des rechnergestützten kausalen Netzwerkmodells verbunden ist, wobei die Differenzaussage von den Aktivitätswerten jedes Knotens in der zweiten Teilmenge von Knoten abhängt, und wobei jeder Aktivitätswert in dem Satz von Aktivitätswerten eine Linearkombination von Aktivitätsmaßen in dem Satz von Aktivitätsmaßen ist; und
(e) Erzeugen, mit der Verarbeitungsvorrichtung unter Verwendung einer Maschinenlerntechnik, eines Klassifikators für den Phänotyp unter Verwendung sowohl des Satzes von Aktivitätsmaßen als auch des Satzes von Aktivitätswerten als Eingaben.

2. Computerisiertes Verfahren nach Anspruch 1, wobei die Schritte (a), (b), (c) und (d) für eine Vielzahl von rechnergestützten kausalen Netzwerkmodellen durchgeführt werden und die Sätze von jedem der rechnergestützten kausalen Netzwerkmodelle entsprechenden Aktivitätswerte zu einer, den Satz von in Schritt (e) durch die Maschinenlerntechnik zu verwendenden Aktivitätswerten, enthaltenden Matrix aggregiert werden.

3. Computerisiertes Verfahren nach Anspruch 2, wobei die Schritte (a), (b), (c), (d) und (e) für eine Vielzahl von rechnergestützten kausalen Netzwerkmodellen durchgeführt werden, und ferner umfassend:

(h1) für jeden Klassifikator, Identifizieren einer oder mehrerer biologischer Entitäten des zweiten Satzes biologischer Entitäten mithilfe von Leistungsstatistiken zur Klassifizierung oberhalb eines Schwellenwerts über die Vielzahl von rechnergestützten kausalen Netzwerkmodellen; und

(h2) Aggregieren aller identifizierten biologischen Entitäten zu einem Satz von Hochleistungsentitäten;

(h3) Erzeugen, durch die Verarbeitungsvorrichtung, eines neuen Klassifikators biologischer Bedingungen basierend auf den mit dem Satz von Hochleistungsentitäten zugewiesenen Aktivitätswerten unter Verwendung einer Maschinenlerntechnik; und

(h4) Ausgabe des neuen Klassifikators.

4. Computerisiertes Verfahren nach einem der Ansprüche 1 bis 3, wobei die Maschinenlerntechnik eine Support-Vector-Machine-Technik beinhaltet.

5. Computerisiertes Verfahren nach einem der Ansprüche 1 bis 4, wobei die Differenzaussage ferner von den Richtungswerten der Kanten zwischen jedem Knoten in der zweiten Teilmenge von Knoten und den Knoten, mit denen jeder Knoten in der zweiten Teilmenge von Knoten verbunden ist, abhängt.

6. Computerisiertes Verfahren nach einem der Ansprüche 1 bis 5, wobei die Linearkombination von Kanten zwischen Knoten in der ersten Teilmenge von Knoten und Knoten in der zweiten Teilmenge von Knoten abhängt und auch von Kanten zwischen Knoten in der zweiten Teilmenge von Knoten abhängt.

7. Computerisiertes Verfahren nach einem der Ansprüche 1 bis 5, wobei die Linearkombination nicht von Kanten zwischen Knoten in der ersten Teilmenge von Knoten abhängt.

8. Computerisiertes Verfahren nach einem der Ansprüche 1 bis 7, wobei das Aktivitätsmaß von Schritt (c) ein Fold-Change-Wert ist und der Fold-Change-Wert für jeden Knoten in der ersten Teilmenge von Knoten einen Logarithmus der Differenz zwischen entsprechenden Sätzen von Behandlungsdaten für die, durch den jeweiligen Knoten dargestellte, biologische Entität darstellt.

9. Computerisiertes Verfahren nach einem der Ansprüche 1 bis 8, wobei die erste Teilmenge von biologischen Entitäten einen Satz von Genen beinhaltet und der erste Datensatz Expressionsniveaus des Satzes von Genen beinhaltet.

10. Computerprogrammprodukt, computerlesbare Befehle aufweisend, die bei Ausführung in einem zumindest einen Prozessor aufweisenden computerisierten System den Prozessor veranlassen, einen oder mehrere Schritte des Verfahrens nach einem der Ansprüche 1 bis 9 durchzuführen.

11. Computerisiertes System, aufweisend eine mit nichtflüchtigen computerlesbaren Anweisungen ausgelegte Verarbeitungsvorrichtung, die bei Ausführung die Verarbeitungsvorrichtung veranlassen, das Verfahren nach einem der Ansprüche 1 bis 9 durchzuführen.

12. Computerisiertes Verfahren nach einem der Ansprüche 1 bis 9, wobei die Optimierung der Differenzaussage das Maximieren der Differenzaussage, das Minimieren der Differenzaussage oder das möglichst genaue Annähern der Differenzaussage an einen Zielwert aufweist.

## Revendications

1. Procédé informatisé de génération d'un classificateur pour une prédiction phénotypique, comprenant les étapes de :

(a) la réception, au niveau d'un dispositif de traitement, de données indicatives d'un modèle de réseau de causalité computationnel, dans lequel les données sont représentatives d'un système biologique qui contribue à un phénotype et inclut :

une pluralité de nœuds qui représentent des entités biologiques dans le système biologique ; et
une pluralité de bords reliant des paires de nœuds parmi la pluralité de nœuds et représentant des relations entre les entités biologiques représentées par les nœuds ;
dans lequel un ou plusieurs bords sont associés à une valeur de direction qui représente une relation d'activation causale ou de suppression causale entre les entités biologiques représentées par les nœuds, et dans lequel chaque nœud est relié par un bord à au moins un autre nœud ;

(b) la réception, au niveau du dispositif de traitement, (i) d'un premier ensemble de données correspondant aux activités d'un premier sous-ensemble d'entités biologiques obtenues dans un premier ensemble de conditions ; et (ii) d'un deuxième ensemble de données correspondant aux activités du premier sous-ensemble d'entités biologiques obtenues dans un deuxième ensemble de conditions différentes du premier ensemble de conditions, dans lequel les premier et deuxième ensembles de conditions concernent le phénotype ;

(c) le calcul, avec le dispositif de traitement, d'un ensemble de mesures d'activité pour un premier sous-ensemble de nœuds correspondant au premier sous-ensemble d'entités biologiques, chaque mesure d'activité représentant une différence entre une première mesure dans le premier ensemble de conditions à partir de b(i) et une deuxième mesure dans le deuxième ensemble de conditions à partir de b(ii) ;

(d) la génération, avec le dispositif de traitement, d'un ensemble inféré de valeurs d'activité pour un deuxième sous-ensemble de nœuds représentant des candidats d'entités biologiques qui contribuent au phénotype mais dont les activités ne sont pas mesurées, en identifiant, pour chaque nœud particulier dans le deuxième sous-ensemble de nœuds, une valeur d'activité qui optimise un énoncé de différence qui représente la différence entre la valeur d'activité du nœud particulier et la valeur d'activité ou la mesure d'activité des nœuds auxquels le nœud particulier est relié par un bord dans le modèle de réseau de causalité computationnel, dans lequel l'énoncé de différence dépend des valeurs d'activité de chaque nœud dans le deuxième sous-ensemble de nœuds, et dans lequel chaque valeur d'activité dans l'ensemble de valeurs d'activité est une combinaison linéaire de mesures d'activité dans l'ensemble de mesures d'activité ; et

(e) la génération, avec le dispositif de traitement utilisant une technique d'apprentissage automatique, d'un classificateur pour le phénotype en utilisant à la fois l'ensemble des mesures d'activité et l'ensemble des valeurs d'activité comme entrées.

2. Procédé informatisé selon la revendication 1, dans lequel les étapes (a), (b), (c) et (d) sont réalisées pour une pluralité de modèles de réseau de causalité computationnel, et les ensembles de valeurs d'activité correspondant à chacun des modèles de réseau de causalité computationnel sont agrégés en une matrice contenant l'ensemble de valeurs d'activité à utiliser à l'étape (e) par la technique d'apprentissage automatique.

3. Procédé informatisé selon la revendication 2, dans lequel les étapes (a), (b), (c), (d) et (e) sont réalisées pour une pluralité de modèles de réseau de causalité computationnel, et comprenant en outre :

(h1) pour chaque classificateur, l'identification d'une ou plusieurs entités biologiques du deuxième ensemble d'entités biologiques avec des statistiques de performance de classification au-dessus d'un seuil à travers la pluralité de modèles de réseau de causalité computationnel ; et

(h2) l'agrégation de toutes les entités biologiques identifiées en un ensemble d'entités hautement performantes ;

(h3) la génération, avec le dispositif de traitement, d'un nouveau classificateur de conditions biologiques sur la base des valeurs d'activité associées à l'ensemble d'entités hautement performantes en utilisant une technique d'apprentissage automatique ; et

(h4) la délivrance en sortie du nouveau classificateur.

4. Procédé informatisé selon l'une quelconque des revendications 1 à 3, dans lequel la technique d'apprentissage machine inclut une technique de machine à vecteur de support.

5. Procédé informatisé selon l'une quelconque des revendications 1 à 4, dans lequel l'énoncé de différence dépend en outre des valeurs de direction des bords entre chaque nœud dans le deuxième sous-ensemble de nœuds et les nœuds auxquels chaque nœud dans le deuxième sous-ensemble de nœuds est relié.

6. Procédé informatisé selon l'une quelconque des revendications 1 à 5, dans lequel la combinaison linéaire dépend de bords entre des nœuds dans le premier sous-ensemble de nœuds et de nœuds dans le deuxième sous-ensemble de nœuds, et dépend également de bords entre des nœuds dans le deuxième sous-ensemble de nœuds.

7. Procédé informatisé selon l'une quelconque des revendications 1 à 5, dans lequel la combinaison linéaire ne dépend pas de bords entre des nœuds dans le premier sous-ensemble de nœuds.

8. Procédé informatisé selon l'une quelconque des revendications 1 à 7, dans lequel la mesure d'activité de l'étape (c) est une valeur de facteur de régulation, et la valeur de facteur de régulation pour chaque nœud dans le premier sous-ensemble de nœuds représente un logarithme de la différence entre des ensembles correspondants de données de traitement pour l'entité biologique représentée par le nœud respectif.

9. Procédé informatisé selon l'une quelconque des revendications 1 à 8, dans lequel le premier sous-ensemble d'entités biologiques inclut un ensemble de gènes et le premier ensemble de données inclut des niveaux d'expression de l'ensemble de gènes.

10. Produit de programme informatique comprenant des instructions lisibles par ordinateur qui, lorsqu'elles sont exécutées dans un système informatisé comprenant au moins un processeur, amènent le processeur à effectuer une ou plusieurs étapes du procédé selon l'une quelconque des revendications 1 à 9.

11. Système informatisé comprenant un dispositif de traitement configuré avec des instructions non transitoires lisibles par ordinateur qui, lorsqu'elles sont exécutées, amènent le dispositif de traitement à effectuer le procédé selon l'une quelconque des revendications 1 à 9.

12. Procédé informatisé selon l'une quelconque des revendications 1 à 9, dans lequel l'optimisation de l'énoncé de différence comprend la maximisation de l'énoncé de différence, la réduction au minimum de l'énoncé de différence ou le fait de rendre l'énoncé de différence aussi proche que possible d'une valeur cible.

**FIG. 1**

EP 2 864 915 B1

200

| RECEIVE BIOLOGICAL DATA<br><br>210 | → | GENERATE SYSTEMS RESPONSE PROFILES (SRPs)<br><br>212 | → | IDENTIFY RELEVANT BIOLOGICAL NETWORKS<br><br>214 | → | GENERATE GENE SIGNATURES BASED ON IDENTIFIED NETWORKS AND SRPs<br>216 |

FIG. 2

EP 2 864 915 B1

302

304

parameter 1

parameter 1

parameter 2

parameter 2

RESPONSE OF
BIOLOGICAL
ENTITY 1

RESPONSE OF
BIOLOGICAL
ENTITY 1

AGENT 1
306

AGENT 2
308

parameter 1

parameter 1

parameter 2

parameter 2

RESPONSE OF
BIOLOGICAL
ENTITY N

RESPONSE OF
BIOLOGICAL
ENTITY N

FIG. 3

EP 2 864 915 B1

400

408

406

402

404

FIG. 4

EP 2 864 915 B1

500

```
┌────────────────────────┐      ┌────────────────────────┐      ┌────────────────────────┐
│  RECEIVE TREATMENT AND │      │   CALCULATE ACTIVITY   │      │ GENERATE ACTIVITY VALUES│
│ CONTROL DATA FOR FIRST │─────▶│ MEASURES FOR FIRST SET │─────▶│ FOR SECOND SET OF      │
│     SET OF ENTITIES    │      │       OF ENTITIES      │      │    ENTITIES            │
│          502           │      │          504           │      │          506           │
└────────────────────────┘      └────────────────────────┘      └────────────────────────┘
                                                                              │
                                                                              ▼
                                                                 ┌────────────────────────┐
                                                                 │ GENERATE NPA SCORES    │
                                                                 │ BASED ON GENERATED     │
                                                                 │ ACTIVITY VALUES AND    │
                                                                 │ NETWORK MODEL          │
                                                                 │          508           │
                                                                 └────────────────────────┘
```

FIG. 5

EP 2 864 915 B1

600

```
┌─────────────────────┐     ┌─────────────────────┐     ┌─────────────────────────┐
│ IDENTIFY A DIFFERENCE│     │  IDENTIFY A DIFFERENCE│    │    COMPUTATIONALLY      │
│      STATEMENT       │ ──▶ │      OBJECTIVE        │──▶ │ CHARACTERIZE THE NETWORK│
│        602           │     │        604            │    │  MODEL BASED ON THE     │
│                      │     │                       │    │  DIFFERENCE OBJECTIVE   │
│                      │     │                       │    │         606             │
└─────────────────────┘     └─────────────────────┘     └─────────────────────────┘
                                                                      │
                                                                      ▼
┌─────────────────────┐                               ┌─────────────────────────┐
│                     │                               │ SELECT ACTIVITIES TO ACHIEVE│
│ OUTPUT ACTIVITY VALUES│ ◀──────────────────────────│  OR APPROXIMATE THE      │
│        612          │                               │  DIFFERENCE OBJECTIVE    │
│                     │                               │         608             │
└─────────────────────┘                               └─────────────────────────┘
```

FIG. 6

700

```
┌─────────────────────┐      ┌─────────────────────┐      ┌─────────────────────┐
│ GENERATE BACKBONE   │      │ GENERATE LEADING     │      │ GENERATE LEADING GENE│
│ OPERATOR BASED ON   │─────▶│ BACKBONE NODE LIST   │─────▶│ NODE LIST USING      │
│ NETWORK MODEL       │      │ USING BACKBONE       │      │ BACKBONE OPERATOR    │
│ 702                 │      │ OPERATOR             │      │ 706                  │
│                     │      │ 704                  │      │                      │
└─────────────────────┘      └─────────────────────┘      └─────────────────────┘
```

FIG. 7

800

RECEIVE CENTERED
EXPRESSION DATA
802

APPLY BACKBONE OPERATOR
TO CENTERED DATA TO
GENERATE ACTIVITY VALUES
804

APPLYING MACHINE LEARNING
ALGORITHM TO ACTIVITY
VALUES
806

FIG. 8

```
┌─────────────────────────────┐
│ FOR i=1:NUMBER OF CANDIDATE  │
│         NETWORKS             │
└─────────────────────────────┘
              │
              ▼
┌─────────────────────────────┐
│     GENERATE BACKBONE        │
│     OPERATOR i BASED ON      │
│      NETWORK MODEL i         │
│            902               │
└─────────────────────────────┘
              │
              ▼
┌─────────────────────────────┐
│            END               │
└─────────────────────────────┘
```

900

┌──────────────────────────┐      ┌──────────────────────────┐
│  AGGREGATE BACKBONE      │      │  CLASSIFY EXPRESSION DATA│
│  OPERATORS TO GENERATE A │  →   │  USING THE CLASSIFIER    │
│  CLASSIFIER KERNEL       │      │  KERNEL                  │
│          904             │      │          906             │
└──────────────────────────┘      └──────────────────────────┘

# FIG. 9

FOR i=1:NUMBER OF CANDIDATE
NETWORKS

1000

GENERATE CLASSIFIER i BASED
ON NETWORK MODEL i
1002

GENERATE STATISTICS FOR
CLASSIFIER i
1004

IDENTIFY BACKBONE NODES IN
NETWORK i WITH STATISTICS
ABOVE THRESHOLD
1006

END

AGGREGATE ABOVE-
THRESHOLD NODES INTO
FEATURE SPACE FOR NEXT
CLASSIFIER
1008

FIG. 10

EP 2 864 915 B1

```
FOR i=1:NUMBER OF CANDIDATE
NETWORKS
```
⟍1100

```
RECEIVE CENTERED
EXPRESSION DATA
1102
```
→
```
APPLY BACKBONE OPERATOR i
TO CENTERED DATA TO
GENERATE ACTIVITY VALUES
1104
```
→
```
SORT Z-SCORES OF ACTIVITY
VALUES BY ORDER OF F-
STATISTIC
1106
```

```
GENERATE P_GS AS MEAN-RANK
ENRICHMENT P-VALUE BASED
ON F-TESTS
1108
```
→
```
GENERATE CUMULATIVE SUM
OF ORDERED VALUES
1110
```
→
```
RECOMPUTE F-TEST FOR EACH
INTERMEDIATE SUM
1112
```

```
SELECT CUMULATIVE SUM JUST
BEFORE F-TEST VALUE
DECREASES
1114
```
→
```
OUTPUT SET OF BACKBONE
NODES CORRESPONDING TO
SELECTED CUMULATIVE SUM
1116
```
→
```
END
```

```
CREATE MATRIX WITH ACTIVITY
VALUES OF BACKBONE NODES
WITH P_GS NOT EXCEEDING A
THRESHOLD
1118
```

FIG. 11

```
┌─────────────────────────────┐
│ FOR i=1:NUMBER OF CANDIDATE │
│          NETWORKS           │
└─────────────────────────────┘
              │
              ▼
┌─────────────────────────────┐              1200
│         FOR b=1:B           │
└─────────────────────────────┘
              │
              ▼
```

| RECEIVE CENTERED EXPRESSION DATA 1202 | → | APPLY BACKBONE OPERATOR i TO CENTERED DATA TO GENERATE ACTIVITY VALUES 1204 | → | SAMPLE ACTIVITY VALUES WITH REPLACEMENT 1206 |

| APPLY A MACHINE LEARNING ALGORITHM TO GENERATE A CLASSIFIER TO THE SAMPLED VALUES 1208 | → | RECORD PREDICTION ERROR EB 1210 | → | END |

```
                                    END
                                     │
                                     ▼
```

| GENERATE AN ENSEMBLE PREDICTOR USING WEIGHTED VOTING BASED ON PREDICTION ERROR 1212 |

FIG. 12

```
┌──────────────────────┐    ┌──────────────────────┐    ┌──────────────────────┐
│  RECEIVE CENTERED    │    │ APPLY BACKBONE       │    │ COMPARE ACTIVITY     │
│  EXPRESSION DATA     │───▶│ OPERATOR TO CENTERED │───▶│ VALUE STATISTICS TO  │
│  1302                │    │ DATA TO GENERATE     │    │ P-VALUE THRESHOLD    │
│                      │    │ ACTIVITY VALUES      │    │ 1306                 │
│                      │    │ 1304                 │    │                      │
└──────────────────────┘    └──────────────────────┘    └──────────────────────┘

┌──────────────────────┐    ┌──────────────────────┐
│ IF NUMBER OF ACTIVITY│    │ APPLYING MACHINE     │
│ VALUES THAT EXCEED   │    │ LEARNING ALGORITHM   │
│ THRESHOLD IS GREATER │───▶│ TO NODES             │
│ THAN Y, ADJUST       │    │ CORRESPONDING TO     │
│ THRESHOLD            │    │ ABOVE-THRESHOLD      │
│ 1308                 │    │ ACTIVITY VALUES      │
│                      │    │ 1310                 │
└──────────────────────┘    └──────────────────────┘
```

FIG. 13

FIG. 14

1500

CPU 1506

COMMUNICATIONS INTERFACE UNIT 1508

INPUT/OUTPUT CONTROLLER 1510

SYSTEM MEMORY

RAM 1502

ROM 1504

STORAGE DEVICES

OPERATING SYSTEM 1512

APPLICATION(S) 1514

DATABASE(S) 1516

FIG. 15

FIG. 16

1702

NPA scores for the fold-changes
Exposure 1 vs exposure 2: 1 positive number
With its 3 companion statistics

$$NPA(\varsigma,\beta) = \frac{1}{|\{x \to y\} \text{ s.t. } x,y \notin V_0|} \sum_{x \to y} (f(x) + sign(x \to y)f(y))^2$$

s.t. $x, y \notin V_0$

$$f_2 = -L_3^{-1}L_2^T f_1 \equiv Kf_1$$

Compute f1=fold changes

Leading nodes / genes

Identify the most important
nodes and genes, that can be
used also with any other
signature/method

If small gene
list needed

| Exposure 2 | Data Matrix |
| Exposure 1 | Gene Expression |

Network N

Compute f1=centered data

$$f_2 = -L_3^{-1}L_2^T f_1 \equiv Kf_1$$

1704

Network signature → Gene signature

Classification by
SVM or any other
⇔ SVM with kernel
$KK^T$
Decision boundary denoted
by D(SVN(N)), K=K(N)

Can be filtered
according to leading nodes

FIG. 17

FIG. 18

* = p-value (w.r.t. expr replicates) < 0.05
*o = p-value (w.r.t. downstream permutations) < 0.05
k* = p-value (w.r.t. backbone permutations) < 0.05

——— 50% downstream
– – – 50% backbone
——— 95% downstream
– – – 95% backbone

FIG. 19

FIG. 20 (part 1)

FIG. 20 (part 2)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Reverse Causal Reasoning Methods - White Paper,* 2011, 1-26
- **WEI KEAT KIM et al.** Master regulators used as breast cancer metastasis classifier. *Pac Symp Biocomput.,* 2009, 504-15
- **PARKINSON et al.** *Nucl. Acids Res.,* 2010
- **MARTIN et al.** *BMC Syst Biol.,* 31 May 2012, vol. 6 (1), 54
- **TIBSHIRANI ; HASTLE ; NARASIMHAN ; CHU.** Diagnosis of multiple cancer types by shrunken centroids of gene expression. *Proc. Natl. Acad. Sci.,* 2002, vol. 99 (10
- **TIBSHIRANI et al.** Diagnosis of multiple cancer types by shrunken centroids of gene expression. *Proc. Natl. Acad. Sci.,* 2002, vol. 99, 6567-6572